# EUROPEAN PATENT SPECIFICATION

(11) **EP 1 874 367 B1**
(45) Date of publication and mention of the grant of the patent: **06.07.2011**
(21) Application number: 06722909.6
(22) Date of filing: 25.04.2006
(51) Int. Cl.: C12N 5/073, C12N 5/079

(54) **BIOCOMPATIBLE MATERIAL FOR SURGICAL IMPLANTS AND CELL GUIDING TISSUE CULTURE SURFACES**
BIOKOMPATIBLES MATERIAL FÜR CHIRURGISCHE IMPLANTATE UND ZELL-FÜHRUNGSGEWEBEKULTURFLÄCHEN
MATÉRIAU BIOCOMPATIBLE POUR IMPLANTS CHIRURGICAUX ET SURFACES DE CULTURE CELLULAIRE DE GUIDAGE DE CELLULES

(30) Priority: 26.04.2005 DK 200500610; 01.07.2005 DK 200500981; 27.04.2005 US 675096 P; 19.07.2005 US 700306 P
(43) Date of publication of application: 09.01.2008
(73) Proprietor: Arhus Universitet, DK-8000 Arhus C (DK)
(72) Inventor: DUCH, Mogens, Ryttergard, DK-8240 Risskov (DK); FOSS, Morten, DK-8660 Skanderborg (DK); PEDERSEN, Finn Skou, DK-8210 Århus V (DK); JUSTESEN, Jeannette Hoffmann Frisch, DK-8000 Aarhus C (DK); ANDERSEN, Lars Klembt, DK-6470 Sydals (DK); CROVATO, Trine Elkjaer Larsen, DK-8210 Århus V (DK); MARKERT, Lotte, DK-8000 Århus C (DK); BESENBACHER, Flemming, DK-8210 Århus V (DK)
(74) Representative: Schwarze, Holm
(86) International application number: PCT/DK2006/000217
(87) International publication number: WO 2006/114098

(56) References cited:
- WO-A-95/12369
- US-A- 5 691 305
- US-A- 5 770 417
- US-A1- 2004 006 396
- US-B1- 6 419 491
- US-B1- 6 767 928
- CHESMEL K D ET AL: "CELLULAR RESPONSES TO CHEMICAL AND MORPHOLOGIC ASPECTS OF BIOMATERIAL SURFACES. II. THE BIOSYNTHETIC AND MIGRATORY RESPONSE OF BONE CELL POPULATIONS" JOURNAL OF BIOMEDICAL MATERIALS RESEARCH, WILEY, NEW YORK, NY, US, vol. 29, September 1995 (1995-09), pages 1101-1110, XP008058327 ISSN: 0021-9304
- S. TURNER ET AL.: "Cell attachment on silicon nanostructures" JOURNAL OF VACUUM SCIENCE AND TECHNOLOGY: PART B., vol. 15, no. 6, 1997, pages 2848-2854, XP002364124 USAVS / AIP, MELVILLE, NEW YORK, NY.

## Description

### Technical field

The present invention provides a biocompatible material having a surface structure and composition that affects a cellular function, in particular cellular functions related to bone cell mineralization and the formation of bone tissue, differentiation, in particular neuronal differentiation, of embryonic stem cells, and/or growth of embryonic stem cells, in particular of undifferentiated embryonic stem cells.

### Background of the invention

The promotion of selected cellular functions is an important task in a variety of applications, such as the development of suitable implants, the productions of undifferentiated stem cells and/or the like. Biocompatible materials, on which living cells can attach, grow, and/or differentiate and/or further perform diverse biological functions, are desirable for a variety of therapeutic purposes.

Degenerative disorders, cancer and trauma of the musculoskeletal apparatus constitute an increasing problem in public health. Spine disorders alone affect 30 percent of the adult population, and 40 percent of those older than 65 years have symptoms of osteoarthritis. More than 1.3 million joint alloplasties are performed annually worldwide to treat debilitating end stage arthritis. Since there are no accepted therapies to prevent osteoarthritis, it is anticipated that the number of arthroplasties performed will rise dramatically over the next several decades, due to the aging of the western population. At present time, more than 25 percent of all health care expenditures in Europe and USA are related to musculoskeletal conditions, and the budgets to treat such disorders in USA (254 Billion USD) are for instance double the resources used for research and teaching in total.

The main surgical treatments of these disorders rely on the use of metallic medical implants in conjunction with bone or bone substitutes. The implants must be successfully incorporated in the bone tissue in order to obtain good clinical results. Major advances and results have been achieved in this area during the last decades, but implant loosening over time continues to be a significant problem for successful long-term joint replacements. The current implant surfaces are not able to bridge larger bone defects and maintain long-term stability alone. The use of bone graft taken from the patients themselves to solve these problems is followed by a high donor site morbidity of 15-30 percent. As many as 20 % of the patients undergoing hip replacement develop bone loss around the prosthesis within 10 to 15 years of the initial surgery, and in spine fusion surgery 20-30 percent of the patients obtain poor fusion. Furthermore, as the near-future patient population will include a significant number of younger patients, the problem concerning long-term aseptic implant loosening is predicted to increase dramatically.

Improvement of implant behavior in bone tissue will therefore have a tremendous impact both in terms of quality of life and economy. The WHO has recognized this by appointing the years 2000-2010 as the "Bone and Joint Decade" (hftp://www.boneiointdecade.org/), an initiative also approved by the Danish Ministry of Health.

The biocompatibility/biointegration of an implant in the body is extremely complicated, involving processes traditionally belonging to medical science, surface science, materials science, and molecular biotechnology. When an implant is placed in tissue, a *race for the surface* starts immediately. Within a few milliseconds after the implant is inserted into the body, a biolayer consisting of water, proteins and other biomolecules from the physiological liquid is formed on the implant surface. Subsequently, cells from the surrounding tissue migrate to the area around the implant due to stimulation by cytokines and growth factors in the biolayer. The interaction between an implant surface and the cells is thus mediated through this biolayer. The properties of the implant surface strongly influence the properties of the layer and this influence needs to be understood and controlled in order to optimize biocompatibility. Of equal importance are the properties of the cells, e.g. their ability to communicate through the extracellular matrix by signal molecules. During bone healing numerous bioactive signal molecules control bone formation and some proteins have shown capability of stimulating bone healing to implants. All these mechanisms contribute to the response of the tissue to the implant and influence whether the implant is successfully anchored with sufficient mechanical strength in the bone of the patient or whether an inflammatory reaction against the implant occurs, which finally will result in aseptic loosening and operative failure.

Biocompatible materials, on which bone tissue cells, can attach, and/or grow, and further perform diverse biological functions, are required for therapeutic purposes, in particular in surgical treatments involving the introduction of implants, such as prostheses and bone substitutes. Achieving a successful outcome by such treatment presents a formidable challenge, since an implant needs to allow tissue regeneration at the implant site while avoiding becoming a target for the body's own powerful rejection mechanisms. The clinical success of an implant depends of the cellular behavior in the immediate vicinity of the interface between an implant and the host tissue. A key element in the progress in this field thus relies on the identification and use of a biocompatible material in the fabrication of these implants.

Bone tissue comprises a number of cell types including osteoprogenitor cells. Marrow stromal cells (MSCs) are pluripotent stem cells that give rise to both osteoprogenitor cells and other cell types. Osteoprogenitor cells can differentiate and form osteoblasts, particularly in response to bone regeneration. Bone modeling proteins (BMP and other growth hormones), produced by the marrow stroma cells, serve to both recruit osteoprogenitor cells and stimulate their maturation into osteoblasts. Osteoblasts secrete e.g. TGF-beta BMP's, other hormones and growth factors etc., which acts both as a chemotactic attractant for osteoprogenitor cells, and stimulates the maturation of osteoblasts and induces the formation of bone matrix. Osteoblasts synthesize and secrete organic bone matrix (like collagen fibers, proteoglycans, osteocalcin, osteonectin and osteopontin) and hence osteoblasts play a key role in the deposition of mineralized bone matrix.

During the mineralization of bone, osteoblasts express alkaline phosphatase, together with a number of cytokines and growth hormones.

In a search for new materials that can be used as a prosthesis and later a scaffold for the regeneration of bone tissue, US 5,282,861 describes reticulated open cell carbon foam, infiltrated with tantalum. Tantalum coated implants are shown to support osseous in-growth in both dental and orthopedic applications. Price et al (J Biomed Matter Res 70:129-138, 2004) report the selective adhesion of osteoblasts on nanophase carbon fibers of smaller diameter, while no effect of fiber surface energy was observed. US 6,767,928 discloses methods of patterning biomaterials in 2D and 3D that are useful for generating 3 dimensional or contoured bioimplants, as well as in cell or tissue culture. In the ongoing development of materials with improved biocompatibility there remains a need to identify materials whose structure is compatible with implant surgery and inductive for bone regeneration.

Swedish patent no. SE 511 863 discloses an implant with a microtextured surface, especially for bone tissue. The implant has its surface formed by illuminating a photoresist layer applied whilst spinning the implant at high speed. This process results in a plurality of spaced-apart depressions distributed over the surface.

US patent no. 6,419,491 discloses a dental implant including a collar section that has an ordered microgeometric repetitive surface pattern in the form of alternating ridges and grooves or depressions.

Nevertheless the above prior art does not solve the problem of identifying the specific structures, if any, that promote selected cell functions, but merely demonstrate a method of making holes of varying sizes in an surface.

Furthermore, during recent years therapeutic uses of embryonic stem cells have attracted considerable attention, and there has evolved an increasing need for efficiently growing neurons and undifferentiated embryonic stem cells as well as the guided, controlled differentiation of embryonic stem cells. Consequently, suitable microenvironments facilitating/promoting these processes are desirable.

### Summary of the invention

Based on the recognition that an individual cell in the body or in a cell culture sees its surrounding tissue or tissue culture surface architecture at the level of micro- and nanostructures, the above and other needs are addressed by providing a biocompatible material or structure with defined surface topography of micro scale features that may be employed in the construction of cell or tissue culture surfaces and/or implants and devices for use in surgical/therapeutic treatment.

Examples of such cells are bone-forming cells. The term bone-forming cells is intended to refer to any kind of cell that is capable of forming bone, including naturally occurring cell types and/or modified cell types, e.g. modified by means of genetic technologies. Other examples include embryonic stem cells and neurons.

The manufacture of a structure having the desired surface topography (that may be entirely artificial or may mimic a surface architecture observed in nature) requires techniques capable of defining features that have micrometer scale or nanometer scale dimensions. The present invention exploits the tools and techniques presently developed within micro- and nano-technology, which allow the design and construction of structures whose surface architecture may have a lateral feature size as small as approximately 6 nm. This feature size can be achieved e.g. by colloidal lithography of ferritin followed by removal of the organic phase leaving behind ion dots. In particular, the use of e-beam lithography and photolithography allows the manufacture of a surface topography which is precisely defined and which can be precisely reproduced in relevant applications.

In particular, it has turned out that when at least a part of a surface of such a biocompatible material is characterized by a micrometer scale topographical structure comprising a plurality of features where at least one lateral dimension of any one of said features is between about 0.1 µm and about 10 µm, a number of cell functions of at least one of a variety of different cell types are significantly improved.

For example, in the context of bone implants, it has turned out that different types of structures have a significantly different effect on the mineralization of bone forming cells as well as the differentiation of embryonic stem cells.

In particular, regular patterns of spaced-apart protrusions that extend out of the surface have been found to be particularly efficient for promoting the above-mentioned cell functions

On one hand, the sizes of the protrusions and the sizes of the gaps between features have been found to be relevant parameters. In particular, each of the protrusions has a cross section with a minimum cross-sectional diameter no larger and preferably smaller than 2 µm, preferably between 0.1 µm and 2 µm, more preferably between 0.5 µm and 2µm, e.g. between 0.1 µm and 1.5µm or between 0.5 µm and 1.5µm. Furthermore, when the protrusions are positioned on grid points of a regular two-dimensional grid where the distance between adjacent grid points along at least one dimension is no larger than 7µm, e.g. smaller than 4µm, e.g. between 0.1 µm and 4 µm, preferably between 0.5 µm and 3.5µm, e.g. between 1µm and 3µm, the promotion of mineralization and other cell functions have been found to be particularly effective. One group of structures where each protrusion has a minimum and maximum cross-sectional diameter between 1 µm and 2 µm and centre-to-centre distances between 3 µm and 8 µm have been found to be particularly effective.

Apart from the feature dimensions, it has further turned out that types of structural patterns are particularly advantageous with respect to the promotion of mineralization and different aspects of the differentiation of embryonic stem cells.

In one embodiment, the structures include protrusions of different cross-sectional geometry, such as round protrusions (e.g. circular or oval) and protrusions having a shape including corners, such as polygons, triangles rectangles, squares, hexagons, stars, parallelograms etc. In particular, when the protrusions of different cross sectional geometry are arranged on a regular two-dimensional grid in an alternating pattern, e.g. in alternating rows, the promotion of mineralization has been found to be particular effective. Likewise, good results have been obtained when a structure includes protrusions of different cross-sectional area, in particular when the protrusions are arranged in regular patterns. One such pattern that has been found to provide good mineralization resembles sharkskin and will be described in more detail herein.

Another type of structures that has provided good results are structures where the protrusions are positioned on grid points of a two-dimensional regular grid such that only a subset of grid points are covered by protrusions, i.e. some grid points are not covered by a protrusion. A similar advantageous effect has been observed when the regular grid is a hexagonal grid. In particular, good results have been achieved when the protrusions are arranged in parallel rows where the centre-to-centre distance between adjacent protrusions is different in adjacent rows. When the centre-to-centre distance between adjacent protrusions in every some rows is an integer multiple of the corresponding distance in the corresponding adjacent rows, flat areas are created surrounded by protrusions. In particular, when the protrusions of the rows with the larger centre-to-centre distances are aligned with the corresponding centers between protrusions of the adjacent rows, so as to be placed the protrusions on the corners of hexagons, these areas have a hexagonal shape which in turn has turned out to be particularly advantageous.

In the context of human or animal embryonic stem cells, it has further turned out that when at least a part of a surface of such a biocompatible material is characterized by a micrometer scale topographical structure comprising a plurality of features where at least one lateral dimension of any one of said features is between about 0.1 µm and about 10 µm, and in particular between about 0.5µm and about 2 µm, the promotion of the growth of undifferentiated embryonic stem cells is significantly improved, when the cells are brought into contact with the surface. This improvement is particularly pronounced when the features are arranged in a regular pattern having a minimum gap size between adjacent/nearest-neighbor features of between about 2µm and about 6µm.

When the structure includes a plurality of elongated ridges arranged in a regular pattern resembling sharkskin as described herein, it has turned out that the promotion of the differentiation of embryonic stem cells is improved, when the cells are brought into contact with the surface.

Likewise, in the context of differentiation of embryonic stem cells, it has further turned out that when at least a part of a surface of such a biocompatible material is characterized by a micrometer scale topographical structure comprising a plurality of features where at least one lateral dimension of any one of said features is between about 0.1 µm and about 10 µm, and in particular between about 0.5µm and about 2 µm, the promotion of the neuronal differentiation of embryonic cells, preferably when the cells are subjected to media supplemented with neuronal growth factors and compounds, is significantly improved, when the cells are brought into contact with the surface. This improvement is particularly pronounced when the features are arranged in a regular pattern having a minimum gap size between adjacent/nearest-neighbor features of between about 2µm and about 6µm. Furthermore, this improvement is particularly pronounced when the features include protrusions regularly arranged so as to generate a pattern where respective pluralities of protrusions are arranged so as to surround a corresponding flat area without protrusions, i.e. an area where the surface area is depressed relative to the top surface of the protrusions. Such a pattern may be provided when the features/protrusions are regularly arranged in alternating rows where the features in adjacent rows are arranged with different pitch distances, e.g. such that the pitch distance in one row is an integer multiple of the pitch distance in the adjacent row, and/or shifted relative to each other. Preferably, at least some of the protrusions have a substantially round, e.g. circular, cross section.

Even further, it has turned out that when at least a part of a surface of such a biocompatible material is characterized by a micrometer scale topographical structure comprising a plurality of features where at least one lateral dimension of any one of said features is between about 0.1 µm and about 10 µm, and in particular between about 1µm and about 10 µm, the outgrowth of neurites from primary neuronal cells in defined directions is promoted, when the cells are brought into contact with the surface. This improvement is particularly pronounced when the features are arranged in a regular pattern having a minimum gap size between adjacent/nearest-neighbor features of between about 2µm and about 6µm.

According to various aspects, the specification provides a biocompatible material, or a medical implant or biocompatible coating for use in the manufacture of an implant comprising such a biocompatible material. The biocompatible material has an exposed surface, wherein the surface has a topographical structure comprising micro scale features along one or more lateral directions. The parameters that define the topography of the biocompatible material of the invention are ones, which enhance mineralization of bone-forming cells attached to its surface and favor biological interactions conducive of mineralization.

According to further aspects, the specification provides a device for culturing tissue or cells, e.g. a tissue culture dish or flask or any other surface that can accommodate cells, the device including an exposed surface for receiving a tissue or cell culture, wherein the surface has a topographical structure comprising micro or nano scale features as described herein selected to support the growth and/or differentiation of neurons or embryonic stem cells, e.g. the growth of embryonic stem cells in an undifferentiated state. Further aspects relate to methods and tools for the production of such devices, such as stamps containing these structures or blueprint of these structures to be used in e.g. hot embossing and/or injection molding for the production of a biocompatible material including said structures.

### Brief description of the drawings:

The invention will be explained more fully below in connection with embodiments and with reference to the drawings, in which:
Fig. 1 shows a top view of an example of a screening tool - a so-called BioSurface Structure Array (BSSA) wafer - for identifying topographical structures that facilitate/enhance cellular functions such as mineralization by bone-forming cells or growth/differentiation of embryonic stem cells or neurons
Fig. 2 shows a cross sectional view of the screening tool of fig. 1 along the line A-B.
Fig. 3 shows a top view of another example of a screening tool.
Fig. 4 shows a top view (Fig. 4a) and a cross-sectional view (Fig. 4b) of a topographical structure comprising alternating trenches of width X (in µm) and ridges of width Y (in µm).
Fig. 5 shows a top view (Fig. 5a) and a cross-sectional view (Fig. 5b) of a topographical structure comprising square holes and a predetermined pitch distance.
Fig. 6 shows a top view (Fig. 6a) and cross-sectional views (Fig. 6b,c) of a topographical structure comprising rectangular holes of dimension X (in µm)xY (in µm) separated with ridges of width X µm.
Fig. 7 shows a top view (Fig. 7a) and a cross-sectional view (Fig. 7b) of a topographical structure comprising pillars of a predetermined dimension and a predetermined pitch distance.
Fig. 8 shows a top view of a topographical structure comprising alternating square holes and pillars.
Fig. 9 shows MC3T3 cells cultured on glass (a) and on a reference surface of a BSSA wafer (b). The actin filaments have been visualized by rhodamin labeled Phalloidine staining.
Fig. 10 shows MC3T3 cells cultured on a tester area having a "D2/4" surface structure. The actin filaments have been visualized by rhodamin labeled Phalloidine staining.
Fig. 11 shows MC3T3 cells cultured on a tester area having a "D2/10" surface structure. The actin filaments have been visualized by rhodamin labeled Phalloidine staining.
Fig. 12 shows schematic top views of examples of topographical structures used for mineralization and/or growth/differentiation of embryonic stem cells or neurons.
Fig. 13 shows examples of topographical structures that promote mineralization.
Fig. 14 illustrates an example of a gene induction assay for use in combination with mineralization inducing genes.
Fig. 15 shows a sample holder for use in a bone-forming assay in sheep.
Fig. 16 shows two examples of views of a section of a BSSA wafer showing parts of four tester squares.
Fig. 17 shows experimental results that illustrate how selected structures increase the number of characteristic embryonic stem cell colonies in a cell culture as compared to a control structure.
Fig. 18 shows experimental results that illustrate how a selected structure resembling sharkskin directs differentiation as compared to a control structure.
Fig. 19 shows quantitative results illustrating how structures within selected size ranges increase the number of characteristic embryonic stem cell colonies in a cell culture as compared to a control structure.
Fig. 20 shows quantitative results illustrating how some selected structures increase the number of characteristic embryonic stem cell colonies in a cell culture as compared to control structures.
Fig. 21 shows quantitative results illustrating how some structures enhance the quality of the embryonic stem cell colonies with respect to the phenotypic appearance of the embryonic stem cell colonies while other structures guide the embryonic stem cells down the differentiation pathway.
Figs. 22-26 show pictures of cells allowed to differentiate for 14 days in B27 medium, fixed, and stained with antibodies against β-tubulin III (neuronal marker, red) and DAPI (which stain cell nuclei, blue) on a control structure and on respective topographical structures.
Fig. 27 shows quantitative results illustrating the degree of neuronal differentiation on different structures.
Fig. 28 shows quantitative results illustrating the degree of neuronal differentiation on different structure sizes.
Fig. 29 shows pictures of primary neuronal culture stained with anti-β-tubulin III antibody on different structures.
Fig. 30 schematically shows a cross-sectional view of a tissue culture dish having an exposed surface with a mirco-scale structure.

### Detailed description of the invention

It is generally desired that implants are successfully incorporated in the bone tissue in order to obtain good clinical results. Major advances and results have been achieved in this area during the last decades, but implant loosening over time continues to be a significant problem for successful long-term joint replacements. An implant or device having a surface that can promote mineralization by bone-forming cells may improve the outcome of treatments based on their use. Thus, the specification provides a biocompatible material or structure, which supports mineralisation by bone-forming cells (including osteoblasts).

Orthopedic implants have a limited lifetime, where poor adhesion between the implant and bone tissue can lead to dislocation of the implant. Thus the specification further provides an implant surface, which supports mineralization of bone-forming cells, thereby improving the biocompatibility of the implant.

Furthermore, in a different application, e.g therapeutic applications, it is desirable to produce a specific cell type e.g. dopaminergic neurons for cell replacement therapies for Parkinson disease. Similarly, for e.g. drug screening purposes, it is desirable to control the reproducibility, quality and amount of a uniform population of a specific cell type, so as to enable/facilitate large scale screening and testing of potential new drugs.

In some applications it is desirable to grow large quantities of embryonic stem cells in an undifferentiated state and to subsequently induce the embryonic stem cells to differentiate into a desired cell type. Once differentiated into a specific cell type these specific cell types may be used for many different applications such as drug screening, cell replacement therapy, diabetes, cartilage damage, etc.

The present invention is based on the recognition that cellular functions that direct mineralization, growth, and/or differentiation are strongly influenced by the cell's microenvironment. Thus, it is thought that mineralization of bone cells *in vivo,* the growth of neurons or undifferentiated embryonic stem cells as well as their subsequent differentiation may depend on the provision of a suitable structure to which the cells can attach. In particular the invention recognizes that the 2- and 3-dimensional architecture, or topography, of surfaces in the microenvironment of a cell, is a critical factor for above processes. There are a myriad of different possible microenvironments. In one aspect, the present invention thus concerns the provision of a biocompatible material whose surface topography creates a specific microenvironment that may enhance mineralization and lead to a better integration of the implant into the remaining bone. Other cell functions that may be influenced by the topography of surfaces include cellular growth, expansion, isolation, migration, differentiation, dedifferentiation, intra- or intercellular organization, etc. As proteins and cells range in size from nano-to micrometer these are relevant length scales for the problem of providing a biocompatible material.

### I. Method of demonstrating the biocompatible properties of and the promotion of cellular functions, e.g. the promotion of mineralization, by the biocompatible material of the invention.

The biocompatible material or structure of the invention may be identified by screening materials with different surface topography using a screening tool / assay that provides different candidate topographical structures.

In particular, a mineralization assay, employing for example Alizarin red staining (Example 2, 3), von Kossa staining (von Kossa, J (1901): "Ueber die im Organismus kuenstlich erzeugbaren Verkalkungen." Beitr Pathol Anat Allg Pathol 29: 163-202), ectopic bone formation (Example 5), and in vivo bone formation/bone ingrowth (Example 6) provides a tool for demonstrating the properties of the biocompatible material of the invention and for selecting suitable topographical structures that promote mineralization.

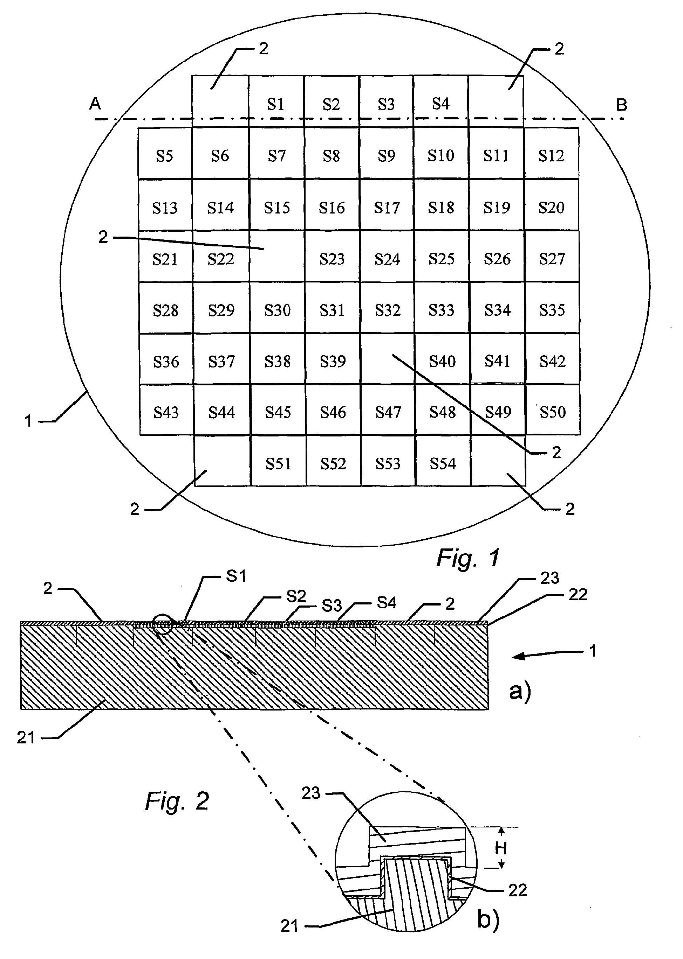

An example of a screening tool suitable for the screening of topographical structures includes a so-called BioSurface Structure Array (BSSA) wafer. Fig. 1 shows a top view of an example of such a biosurface structure array wafer. The BSSA wafer 1 comprises 60 tester areas. A number of tester areas 2 are left "blank", i.e. they have not been processed to have a structured surface. Consequently, the surfaces of the tester areas 2 are substantially flat. Consequently a control experiment is inherently included in each parallel screening test with the BSSA screening tool. The remaining tester areas, designated S1,S2...,S54, comprise respective structured surfaces as described herein. The tester areas are squares of dimension 10mm x 10mm.

A wafer for use as a screening tool to identify structures that induces/enhances cellular functions such as mineralization, growth and differentiation may be manufactured by a number of production techniques.

Examples of procedures for its manufacture include one or more of the following techniques that are known as such in the art:
- Photolithography methods: Photolithography is a process known as such in which geometric shapes/patterns are transferred from a photomask to the surface to be structured, e.g. the surface of a wafer. Photolithography equipment with minimum lateral feature sizes ranging from around 1 micrometer to below 100nm is known as such. Photolithography processes are described in e.g. S.M.Sze: Semiconductor Devices, Physics and Technology, 2nd Edition, John Wiley & Sons 2002, Chapter 12: Lithography and Etching; and in Plummer, Deal, Griffin: Silicon VLSI Technology, Fundamentals, Practice, and Modeling, Prentice Hall 2000, Chapter 5: Lithography.
- E-beam lithography: In principle, E-beam lithography can be used to expose a photoresist in exactly the same way as the light is used in photolithography. E-beam lithography has a particularly high resolution up to around 5nm.
- Hot embossing: Hot embossing uses a master stamp to imprint micro- and nanometer scale structures on polymer substrates. The method allows the master stamp to produce many fully patterned substrates using a wide range of polymer materials. Hot embossing provides a low-cost, highly versatile manufacturing method that is well suited for the manufacture of BSSA for uses ranging from research and development applications to high-volume production. High aspect ratios with a very high degree of homogeneity may be achieved for micro- and nanometer scale structures on large-sized wafers, such as 8 inch or 12 inch wafers. Features sizes below 20nm are possible. The master stamp may be produced by e.g. E-beam lithography techniques.
- Other examples of production steps or processes that may be involved in the production of the biocompatible material or structure include nano imprint lithography, laser ablation, chemical etching, plasma spray coating, abrasive blasting, engraving, scratching, micro machining, or the like.

Fig. 2 shows a cross sectional view of the wafer of Fig. 1. Fig. 2a shows a cross section of the entire width of the wafer along the line labelled A-B. Fig. 2b shows an enlarged view of a portion of the surface of one of the tester areas. The wafer 1 has a layered structure including a patterned substrate layer 21, e.g. a silicon layer, and a surface layer 23. The surface of the wafer is patterned, e.g. by a photolithography process, to provide different patterns on the surfaces of the respective tester areas S1, S2, ..., S54. The structures have a depth/height H. In a photolithography process the height H is controllable by the etching process. The patterned surface is covered by a thin layer of silicon dioxide 22, and/or a surface layer 23 of a different biocompatible material such as tantalum or any other metal, metal oxide, metal nitrides, metal carbides, diamond, diamond like carbon, semiconductor, semiconductor oxide/nitride, insulator, polymers, copolymers. Between the tester areas there is a "blank" border area with no structure, i.e. the border area has not been processed to have a structured surface. In this case the width of the blank border area is 0.3 mm. A blank border line aids visual alignment and identification of the structures. The blank border further serves as a small control surface next to each tester square.

It is noted that the fig. 2 is schematic and not drawn to scale. In particular, the vertical dimensions may be exaggerated to improve readability.

### II. Chemical composition of a biocompatible material

Embodiments of a biocompatible material or structure may take a variety of forms, such as
- a medical implant or a biocompatible coating for use in the manufacture of a medical implant,
- a tissue culture dish/flask having a surface to be exposed to the cell culture, where the exposed surface with structures supporting the desired cellular function, e.g. growth or differentiation of neurons or embryonic stem cells in an undifferentiated state,
- a tissue culture surface or e.g. tissue culture plastic that has been modified to display these structures or blueprint of these structures on the surface. In this respect tissue culture plastic means any polymers that can be used to produce a surface that can be used for growth of cells in vitro in cell culture.

Embodiments of a biocompatible material or structure may comprise a substrate layer, and optionally, a surface layer.

Suitable base materials for the preparation of the biocompatible material or structure include any semiconductor (doped or not-doped), a single metal, a metaloxide, a metal nitride, an alloy, a ceramic, a polymer, a co-polymer, a composite, a drug delivery system, a polymer with bioactive molecules, other bioactive compounds or any combination thereof.

In embodiments of the specification, the surface layer comprises a material that is sufficiently biocompatible to enhance the mineralisation of bone-forming cells. Examples of surface layers include a metallic surface deposit, e.g. tantalum, titanium, Ti-Al-V alloys, gold, chromium, metal oxides, semiconductor oxides, metal nitrides, semiconductor nitrides, polymers, biopolymers, or other alloys. Preferred surface compositions for implants include tantalum or titanium.

In some embodiments, the biocompatible material or structure comprises additional components such as one or more bioactive compound, which may be deposited or adsorbed on the exposed surface or surface layer of said material or structure. For example, said compound may be selected from the group consisting of an antibody, antigen, glycoprotein, lipoprotein, DNA, RNA, polysaccharide, lipid, growth hormone, organic compound, and inorganic compound. Preferably, a growth hormone selected from the group consisting of BMP, EGF-like, TGF-beta is adsorbed or bound to the surface of the biocompatible material.

### III. Structural properties of a biocompatible material

All or part of the surface of the biocompatible material or structure (which may take a variety of forms as described above) comprises micrometer scale features in one or more dimensions within the plane defined by the surface of the material or structure. The terms micro scale and micrometer scale as used herein are intended to refer to a length scale in the range of between about 1µm and about 1000µm. The term nanometer scale as used herein is intended to refer to a length scale in the range of between about 1 nm and about 1000nm, in particular between about 1 nm and about 100nm.

In embodiments of the specification the features are structural/topographical features such as protrusions extending out of the surface of the biocompatible material.

In embodiments of the specification a micrometer scale feature has a lateral dimension in at least one lateral direction, where said dimension is selected from one of the intervals: between about 1 µm and about 20 µm; between about 1-10µm; between about 10-20µm, between about 1µm -2 µm, between about 2µm -4 µm, between about 4µm - 6 µm, between about 6 µm -8 µm, between about 8µm -10 µm; between about 10-12µm; between about 12-14µm; between about 14-16µm; between about 16-18µm; between about 18-20µm. Preferably at least one lateral dimension is between 1µm -6 µm. Hence, in embodiments of the invention, the shortest distance from any given point within the cross-sectional area of a feature to the edge of the cross-sectional area is less than 20 µm, less than 10 µm, less than 8 µm, less than 6µm, less than 4 µm, e.g. less than 2µm.

The lateral dimension is measured in a direction substantially parallel to the surface or at least substantially tangential to the surface.

The maximum distance, or gap, between any micrometer scale feature and its nearest neighbor has lateral dimension in at least one lateral direction where said dimension is selected from one of the intervals: between about 0.5 µm -1 µm, between about 1 µm - 2 µm, between about 2 µm -4 µm, between about 4 µm - 6 µm, between about 6 µm - 8 µm, between about 8 µm -10 µm, between about 10 µm - 12 µm, between about 12 µm - 14 µm, between about 14 µm - 16 µm. Preferably the lateral dimension is between 1 µm - 12 µm. The disposition of micrometer scale features at the surface of the biocompatible material is preferably periodic along one or more lateral direction, and may be described by a periodic function having a lateral pitch dimension selected from one of the intervals: between about 1 µm -2 µm, between about 2 µm -4 µm, between about 4 µm -6 µm, between about 6 µm -10 µm, between about 10 µm -16 µm, between about 16 µm -20 µm, between about 20 µm -24 µm. Preferably the pitch dimension is between 2 µm -12 µm.

The periodic function of the micrometer scale features may have a smaller period along one direction and a larger period, e.g. by a factor of 2, 3,10 or larger, in another direction. Any one micrometer scale feature at the surface of the biocompatible material may be defined as a period of the periodic structure. Hence, the lateral dimensions of a feature of a periodic structure may be defined as the period of the periodic shape/function, i.e. the length of the shortest interval over which the structure repeats its shape.

The depth/height of the micrometer scale features, i.e. their linear dimension in a direction projecting out of the surface of the biocompatible material may be on the nano- or micrometer scale, i.e. the structures may have heights/depths in the range 1 nm - 10µm, or in a range selected from the intervals: of between about 0.07 µm - 0.6 µm, 0.6 µm -1.6 µm, of between about 1.6- 3.0 µm, of between about 3 µm - 10 µm. In some embodiments the structures have a depth/height of greater than 0.6 µm, of at least 1.2 µm, 1.6 µm, 2.0 µm, 3.0 µm, 5.0 µm, 10 µm, even though larger heights such as of at least 20 µm, 50 µm, 100 µm or 1000 µm are possible In some embodiments, all features have substantially the same height, while in other embodiments the features may have different heights.

The lateral cross section of any one micrometer scale feature is preferably geometrical, such as square, rectangular, hexagonal, polygonal or star-shaped. The top and/or side surfaces of the feature are preferably substantially flat. The surfaces of the micrometer scale features can, however, also include features on the nanoscale to achieve a synergistic effect of the topography both on the micrometer and nanometer scale. This can be obtained by e.g. chemical etching (e.g. by NaOH or citric acid), ion etching, colloidal lithography (e.g. by polystyrene beads, bucky balls or proteins), grazing incidence Physical Vapour Deposition coating, CVD coating, or plasma spraying. The features at the surface of the biocompatible material may have the same or different shapes. Preferably the features at the surface of the biocompatible material are geometric (e.g. square, rectangular, hexagonal, star-shaped, or polygonal) in shape.

In some embodiments, the cross-sectional shape of the features may be derived from a simple geometric shape, such as a square, a circle, or the like, e.g. by modifying the corners of a square. Examples of such modifications include the cutting off and/or rounding off of corners. Hence, such shapes are generally square, circular, or the like, but they deviate slightly from a perfect square or circular shape, thereby introducing additional corners and/or modifying the angles between the edges that meet at each corner.

In general, a 2-dimensional periodic structure may be defined by a unit cell in the plane of the surface having a predetermined shape, such as square, rectangular, hexagonal etc., and a repeat unit defining the detailed structure (the base) in the unit cell, such as holes or protrusions, e.g. square pillars, polygonal pillars, circular pillars, pyramids etc. The positions defined by that unit cell define the repeat distances, while the repeat unit defines the predetermined shape and size. These unit cell positions may be defined by respective 2-dimensional vectors. The grid structure thus results from a translation of the unit cell along the two dimensions defined by the surface, in particular respective multiples of the unit cell dimensions. In one embodiment, the centre position of each feature may be defined by a vector **v** = **n**₁ **v**₁ + **n**₂ **v**₂, where **v**₁ and **v**₂ are linearly independent vectors in the surface and n₁ and n₂ are integers.

In some embodiments, the center of each feature is placed on a grid point of a 2-dimensional grid, e.g. a hexagonal, a rectangular or a square grid with predetermined grid constants.

In some embodiments, all features cover all grid points of such a grid, while in other embodiments not all grid points of the underlying grid are covered. For example, in some embodiments, in every other row of grid points, every other grid point may be covered by a feature. In yet other embodiments, in every second, third, fourth or higher order row, every second, third, fourth, or higher order grid point is left empty.

In some embodiments, the topographical structure may include a plurality of different features, e.g. a number of different features arranged in a regular, e.g. periodic, pattern, e.g. as alternating rows of two, three, or more different features. Examples of such patterns include structures comprising features with square cross-sections and features with circular cross-sections that are arranged in alternating rows.

In some embodiments, the features are arranged in lines and/or rows. In some embodiments, the features in each row have the same pitch distance, while in other embodiments the pitch distance may vary throughout a row and/or from row to row. Similarly, the row-to-row distance may be the same for all rows or vary from row to row. In some embodiments, some or all structures in a row may be rotated with respect to their respective neighbor(s) in the same row. In some embodiments, some or all structures in a row may be rotated with respect to their respective neighbor(s) in the neighboring row(s).

In some embodiments, the lateral dimension of the features in all lateral directions is between 1 µm and about 10 µm. Examples of such features include protrusions with generally square or circular cross sections. In other embodiments the lateral dimension of the features in one direction is between 1 µm and about 10 µm, while the lateral dimension in another direction is larger.

Examples of such features include elongated ridges, ribs, or wells. The side faces of the ridges may be substantially smooth or they may include additional features, e.g. a regular sequence of protrusions and/or recesses. Hence, in some embodiments such ridges may have an appearance that resembles a row of squares, circles or the like that are merged/interconected with their respective neighbours to form an uninterrupted ridge.

In particular, in some embodiments the topographical structure comprises both features with lateral dimensions between 1 µm and about 10 µm in all lateral directions and features with lateral dimensions between 1 µm and about 10 µm in only one direction. Examples of such structures include rows of generally square-shaped and/or circular features where the rows are separated by elongated ridges.

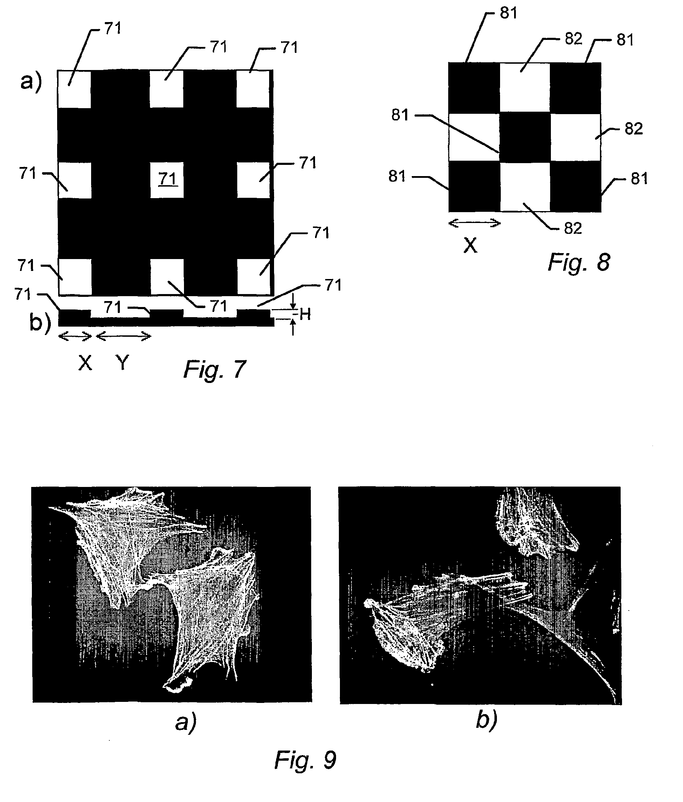

A preferred biocompatible material of the specification has the microstructure D2/4 having a surface characterized by a topography comprising: a two-dimensional periodic structure of square pillars of dimension 2 µm x 2 µm and pitch distance of 6 µm (fig. 7). The depth/height of this dimensional periodic structure is greater than 0.6 µm, and is preferably a vertical dimension of between 1.2 µm and 10.0 µm, and more preferably a vertical dimension of at least 1.2 µm, 1.6 µm, 2.0 µm, 3.0 µm, 5.0 µm, 10 µm, even though larger heights such as of at least 20 µm, 50 µm, 100 µm or 1000 µm are possible.

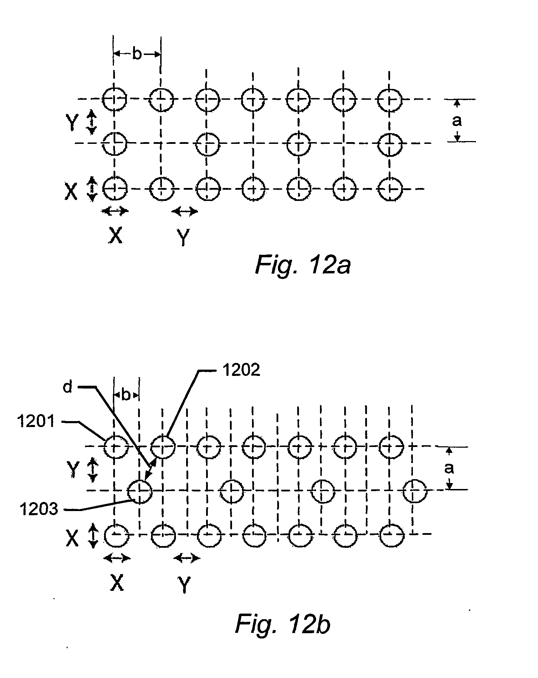

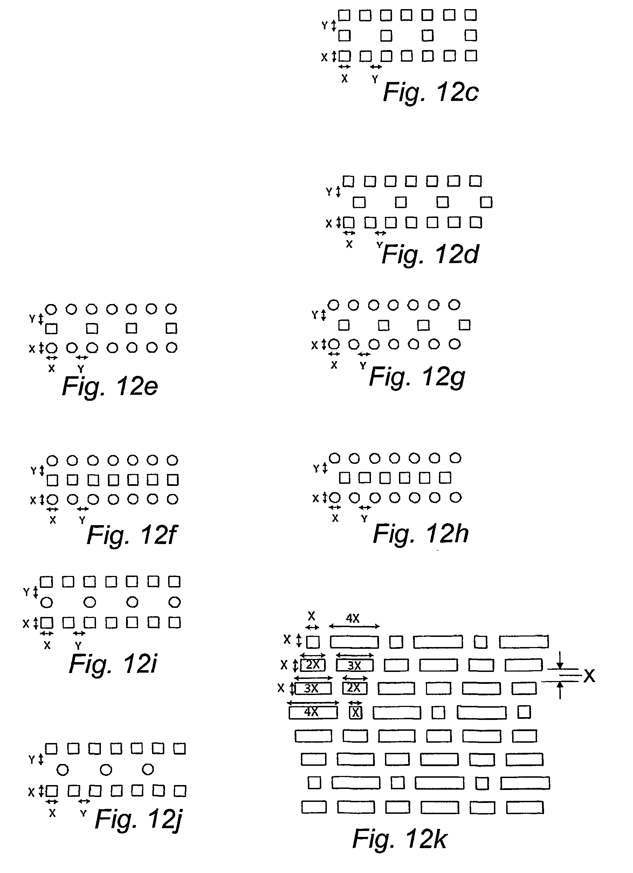

Figs. 12 a-k show top views of examples of the topographical structures with features in the form of protrusions/pillars having a generally circular, square or rectangular cross-section. Each feature has a lateral diameter X in at least one direction, and the gap distance between features in adjacent rows and columns is denoted Y. In figs. 12 a, c, e, f, and i, Y is equal to the gap size between any feature and its nearest neighbor, corresponding to a pitch distance X+Y. In figs. 12 b, d, g, h, and j, the gap distance to the nearest neighbor is different for different features, as exemplified by features 1201, 1202, and 1203 of fig. 12b. Feature 1201 has feature 1202 as its nearest neighbor; consequently the gap size is Y. However, feature 1203 has features 1201 and 1202 as its nearest neighbors with a slightly different gap size d. Accordingly, in figs. 12 b, d, g, h, and j, the pitch distances are different from row to row. In the row including feature 1201, the pitch distance is X+Y, while the pitch distance in the row including feature 1203 is 2(X+Y).

Even though other heights are possible, the structures used in the examples below had a feature height of 1.6 µm unless mentioned otherwise.

In the examples of fig. 12, the center of each feature is placed on a corresponding grid point of a 2-dimensional rectangular grid with grid constants a and b, as illustrated in figs. 12 a and b. However, in figs. 12 a-e, h-i not all of the grid points are actually covered by features, while in figs. 12 f and k all grid points are covered by features. In figs. 12 a, c, e, f, and i, the grid is a square grid with grid constant a=b=(X+Y). In figs. 12 b, d, g, h, and j, the grid is rectangular and the grid constants are a=X+Y and b= (X+Y)/2. In fig. 12 k, the grid constants are a=2·X and b=3.5·X. For selected values of X and Y, wafers have been produced according to figs. 12 a-h where (X,Y) in µm were selected from (X,Y) = (1,1), (1,2), (1,4), (1,6), (2,1), (2,2), (2,4), (2,6), (4,1), (4,2), (4,4), (4,6), (6,1), (6,2), (6,4), (6,6). For selected values of X and Y, wafers have been produced according to fig. 12 k, where X in µm was selected from X= 1, 2, 3, 4, 5, 6, 7, 8. Accordingly, the grid constants a and b of the underlying grids were a = b= 2-12µm for the square grids of figs. 12 a, c, e, f, and i. For the rectangular grids of figs. 12 b, d, g, h, and j, the grid constants in direction a were in the interval between 2 -12µm, the grid constants in direction b were in the interval between 1 - 6µm. For the grid of fig. 12 K, the grid constant b lies in the interval between 3.5 -28 µm and grid constant a lies in the interval between 2 -16µm.

For the purpose of identifying the above structures for different values of X and Y respectively, structures as shown in fig. 12a are referred to as AX.Y in the present description, where X and Y refer to the dimensions X and Y described above. Hence, structure AX. Y includes protrusions/pillars having a circular cross-section of diameter X µm. The protrusions are arranged in parallel rows, where the gap size between adjacent protrusions in every second row is Y µm, while the gap size between protrusions in the remaining rows is (2*Y*+*X*) µm. The gap size between protrusions of adjacent rows is *Y* µm. The protrusions in adjacent rows are aligned with each other.

Similarly, structures as shown in fig. 12b are referred to as B*X*.*Y*. Hence, structure B*X*.*Y* includes protrusions/pillars having a circular cross-section of diameter X µm. The protrusions are arranged in parallel rows, where the gap size between adjacent protrusions in every second row is Y µm, while the gap size between protrusions in the remaining rows is (2*Y*+*X*) µm. The gap size between protrusions of adjacent rows is *Y* µm. The protrusions in the rows having a gap size of (2*Y*+*X*) µm are aligned with the centre of the gaps between protrusions of their respective adjacent rows.

Structures as shown in fig. 12c are referred to as CX.Y. Hence, structure CX.*Y* includes protrusions/pillars having a square cross-section of linear dimension of X µm. The protrusions are arranged in parallel rows, where the sides of the squares are aligned with the direction of the rows, and where the gap size between adjacent protrusions in every second row is Yµm, while the gap size between protrusions in the remaining rows is (2*Y*+*X*) µm. The gap size between protrusions of adjacent rows is *Y* µm. The protrusions in adjacent rows are aligned with each other.

Structures as shown in fig. 12d are referred to as D*X*. *Y*. Hence, structure DX. Y includes protrusions/pillars having a square cross-section of linear dimension of X µm. The protrusions are arranged in parallel rows, where the sides of the squares are aligned with the direction of the rows, and where the gap size between adjacent protrusions in every second row is Y µm, while the gap size between protrusions in the remaining rows is (2*Y*+*X*) µm. The gap size between protrusions of adjacent rows is *Y* µm. The protrusions in the rows having a gap size of (2*Y*+*X*) µm are aligned with the centre of the gaps between protrusions of their respective adjacent rows.

Structures as shown in fig. 12e are referred to as E*X*.*Y*. Hence, structure EX.Y includes protrusions/pillars having a circular cross-section of diameter X µm as well as protrusions/pillars having a square cross-section of linear dimension of X µm. The protrusions are arranged in alternating parallel rows with circular protrusions in every second row, and square protrusions in the remaining rows. The gap size between adjacent protrusions in the rows with circular protrusions is *Y* µm, while the gap size between the square protrusions in the remaining rows is (2*Y+X*) µm. The gap size between protrusions of adjacent rows is *Y* µm. The protrusions in adjacent rows are aligned with each other.

Structures as shown in fig. 12f are referred to as F*X.Y.* Hence, structure F*X.Y* includes protrusions/pillars having a circular cross-section of diameter X µm as well as protrusions/pillars having a square cross-section of linear dimension of X µm. The protrusions are arranged in alternating parallel rows with circular protrusions in every second row, and square protrusions in the remaining rows. The gap size between protrusions within each row and between adjacent rows is *Y* µm. The protrusions in adjacent rows are aligned with each other.

Structures as shown in fig. 12g are referred to as GX.Y. Hence, structure GX.Y includes protrusions/pillars having a circular cross-section of diameter X µm as well as protrusions/pillars having a square cross-section of linear dimension of X µm. The protrusions are arranged in alternating parallel rows with circular protrusions in every second row, and square protrusions in the remaining rows. The gap size between adjacent protrusions in the rows with circular protrusions is *Y*µm, while the gap size between the square protrusions in the remaining rows is (2*Y*+*X*) µm. The gap size between protrusions of adjacent rows is *Y* µm. The square protrusions in the rows having a gap size of (2*Y*+*X*) µm are aligned with the centre of the gaps between the circular protrusions of their respective adjacent rows.

Structures as shown in fig. 12h are referred to as H*X*.*Y*. Hence, structure HX. Y includes protrusions/pillars having a circular cross-section of diameter X µm as well as protrusions/pillars having a square cross-section of linear dimension of X µm. The protrusions are arranged in alternating parallel rows with circular protrusions in every second row, and square protrusions in the remaining rows. The gap size between protrusions within each row and between adjacent rows is *Y* µm. The protrusions in adjacent rows are aligned with each other. The square protrusions are aligned with the centre of the gaps between the circular protrusions of their respective adjacent rows.

Structures as shown in fig. 12i are referred to as I*X*.*Y*. Hence, structure I*X*.*Y* includes protrusions/pillars having a circular cross-section of diameter *X* µm as well as protrusions/pillars having a square cross-section of linear dimension of X µm. The protrusions are arranged in alternating parallel rows with circular protrusions in every second row, and square protrusions in the remaining rows. The gap size between adjacent protrusions in the rows with square protrusions is Y µm, while the gap size between the circular protrusions in the remaining rows is (2*Y*+*X*) µm. The gap size between protrusions of adjacent rows is *Y* µm. The protrusions in adjacent rows are aligned with each other.

Structures as shown in fig. 12j are referred to as J*X*.*Y*. Hence, structure J*X*.*Y* includes protrusions/pillars having a circular cross-section of diameter X µm as well as protrusions/pillars having a square cross-section of linear dimension of X µm. The protrusions are arranged in alternating parallel rows with circular protrusions in every second row, and square protrusions in the remaining rows. The gap size between adjacent protrusions in the rows with square protrusions is *Y* µm, while the gap size between the circular protrusions in the remaining rows is (2*Y*+*X*) µm. The gap size between protrusions of adjacent rows is *Y* µm. The circular protrusions in the rows having a gap size of (2*Y*+*X*) µm are aligned with the centre of the gaps between the square protrusions of their respective adjacent rows.

Hence, in the above examples, the minimum gap size between nearest-neighbor features is *Y* µm, and the minimum centre-to-centre distance between nearest-neighbour features is *X*+*Y* µm.

Structures as shown in fig. 12k are referred to as KX. Structure KX comprises groups of elongated protrusions/ridges of rectangular cross section. The ridges have different lengths and are arranged parallel with each other. The ridges of each group are arranged to form a rectangular (?) shape, such that each group includes a longest ridge as a central ridge. On each side of the central ridge are arranged a series of ridges becoming progressively shorter with increasing distance from the central ridge. The rectangular shape KX includes thus a sequence of ridges of lengths *X* µm, 2*X* µm, 3*X* µm, 4*X* µm, 3*X* µm, 2*X* µm, *X* µm. The width of the ridges is *X*µm. The distance between ridges is *X*µm. The groups of ridges are arranged in a predetermined pattern, such that the ridges are placed along rows, where each row includes ridges of two alternating lengths: A first type of rows includes alternating ridges of length *X* µm and 4 *X* µm., A second type of rows includes alternating ridges of length 2 *X* µm and 3 *X* µm. The overall pattern of ridges resembles a sharkskin structure.

Other preferred structures will be described in connection with the examples below.

### IV. An implant comprising the biocompatible material

According to one aspect, the specification provides a medical implant for use in bone tissue implantation and the like, wherein at least a part of the surface of the implant is characterized by the biocompatible material of the invention, whose surface is characterized by a defined periodic micrometer scale topographical structure that is biocompatible with bone-forming cells, and whose topographical structure is described above under section II and in the examples. A medical implant of the invention includes a dental implant, an orthopedic prothesis/ implant, a spinal implant, a bone substitute that may be contemplated for use in the treatment of a bone fracture, a degenerative disorder, trauma, and cancer.

In a preferred embodiment the entire exposed surface area of the implant, or the biocompatible coating for use in the manufacture of an implant, is composed on a biocompatible material of the invention having a topographical structure that enhances mineralization in bone-forming cells. In an alternative embodiment, one or more parts of said exposed surface area is composed as a biocompatible material of the invention having a topographical structure that enhances mineralization in bone-forming cells. Hence by selectively providing a device with a suitable surface structure, it may be controlled, which parts of the surface should perform in a certain fashion (e.g. mineralization). Other parts of the implant may be formed by other types of structures that could enhance the biocompatibility of e.g. chrondrocytes, epithelial cells where the implant is to be in contact with alternating types of tissue. A surface for rejection of bacteria growth may also be included. As an example a dental implant could be considered. This implant is to consist of 5 different surfaces in order to fulfill the requirements for alternating environments: 1. Optimal for mineralization/bone formation/ingrowth, 2. Optimal biocompatibility for connective tissue (fibroblasts), 3. Optimal biocompatibility for epithelium (epithelial cells), 4. Surface for bacterial rejection, and finally, 5. Optimal surface for the addition of an artificial tooth.

### V. Method of synthesizing a biocompatible topographically modified surface over a contoured, 3D surface of an implant.

An implant surface that is biocompatible for bone-forming cells and enhances mineralization of bone-forming cells, may be manufactured by a number of production techniques, e.g. one or more of the following techniques:
- Die imprinting: By using hard molds (e.g. of SiC or SiN) it is possible to produce patterns directly in other hard materials, like implant metals, by imprinting. The die, which is the master, is typically produced by a combination of e-beam lithography and Reactive Ion Etching. It has been shown that large arrays of nanostructures with width down to 40 nm can be printed in soft metals like Aluminium (S. W. Pang, T. Tamamura, M. Nakao, A. Ozawa, H. Masuda, J. Vac. Sci. Technology B 16(3) (1998) 1145. More specifically, it is desirable to generate die patterns in very hard materials like SiC or SiN when pressing in other hard substrates like Al, Ti, Titanium alloys, stainless steels, Ta, etc - otherwise the die will be damaged or even destroyed. The die imprinting can of course also be applied in softer materials like polymers. Since materials like SiC or SiN are difficult to dry-etch it is desirable to create an etch-mask consisting of e.g. Cr instead of just a photoresist. The mask can be produced in the following way: The lateral pattern is created by e-beam lithography in a resist followed by development, typically in an organic solvent like acetate. This leaves a resist pattern on the surface. The resist pattern is covered by a PVD - deposition of an approximately 100 nm Cr layer and at last lift-off by dissolving the resist using a standard resist remover. Dry etch of the hard die material can be carried out in a Reactive Ion Etching system. The depth of the structure is controlled by ion etching time. At last the Cr mask can be removed in cerium nitrate aquous solution. Now the hard die is ready for imprinting in the surface for synthesizing a biocompatible topographically modified surface. The die can press micro- and nanopatterns in selected areas of the biomaterial by hydraulically pressing the die into selected areas of the surface. The pressure applied will typically be several tonnes for 10-30 seconds. Several areas can be patterned by consecutively patterning areas of the die size. The die size is typically from 10x10 mm² up to 40x40 mm². This micro- and nanoprinting method is highly suitable for patterning selected areas on a contoured 3D implant produced by e.g. Ti, Titanium alloys, tantalum, or stainless steels. But it can of course also be applied to less hard materials like polymeric materials/coatings.
- Imprinting by rolling a die. The method is basically the same as die imprinting, however, here the die is not flat but typically a cylinder. This die-roller is micro- and nanostructured by photolithography or e-beam lithography/Reactive Ion etching as described for the die imprinting above. The setup needs to be modified in order to take into account the curved surface. The die-roller can now be pressed on and rolled over selected areas of the biomaterial by hydraulically pressing the roller-die onto the surface of the implant, thereby imprinting the micro and/or nanostructure. Also here, the implant material can be hard like Ti, Ti-alloys, tantalum or stainless steel, but it does not have to be, so the method is also applicable for e.g. polymers.
- Patterning by colloidal lithography: Here, it is possible to nano-pattern surfaces by depositing colloidal particles (e.g. polystyrene or the protein ferritin), which assemble in a short-range ordered pattern. These particles can e.g. be used as: an etching mask making pillars, a topographical template for making protrusions on the surface, or deposition of e.g. a nanometer metal cluster (e.g. by the metallic center of ferritin).
- Laser patterning by ultra-short laser pulses: This technique can be utilized for high-precision patterning. In particular, the strong nonlinearity of the ablation process leads to a well-defined threshold for material removal, and this has been used to demonstrate the formation of structures even below the diffraction limit (P. P. Pronko, S. K. Dutta, J. Squier, J. V. Rudd, D. Du, G. Mourou: Optical Communication 114, (1995) 106).
- The laser patterning by ultrashort laser pulses can also be used in combination with pre-deposition of quartz spheres (K. Vestentoft, J. A. Olesen, B. H. Christensen, P. Balling: Appl. Phys A 80, (2005) 493 to create large arrays of nanometer-sized holes. More specifically, a layer of quartz spheres is deposited on the surface typically, but not necessarily, creating a densely packed array. By scanning an unfocused laser beam of ultrashort pulses across the surface with the quartz spheres, it is possible to generate large areas of structures in parallel, since the spheres act as individual lenses focusing the laser beam.,
- Laser scanning-beam Interference Lithography: This low-cost method - can be used for fabricating periodic and quasi-periodic and spatially coherent patterns over large surface areas. The methods utilize the interference between two or more coherent planar wave-fronts. (S. Kuiper, H. van Wolferen, G. van Rijn, Journal of Micromechanics and Microengineering 11(1), (2001) 33.

### VI. A device for culturing tissue or cells including an exposed surface having a microscale surface structure

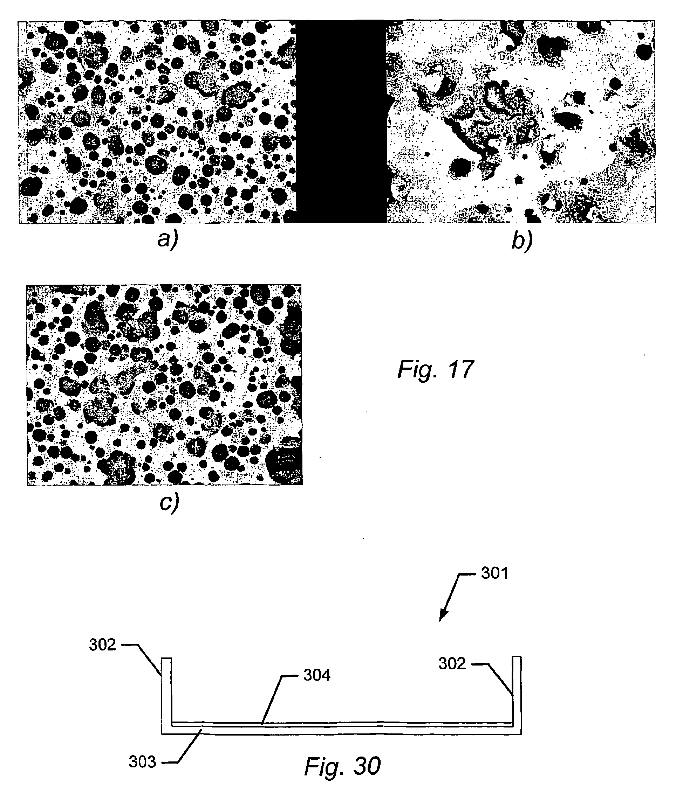

Fig. 30 schematically shows a cross-sectional view of a tissue culture dish having an exposed surface with a mirco-scale structure. The dish 301 comprises an upwardly open receptacle having a bottom 303 and sidewalls 302. In use, the upper surface 304 is exposed to the cell culture or tissue and thus provides a microenvironment for the culture. In embodiments of the invention, the exposed surface 304 has a microscale topographical structure that is selected to promote a predetermined cellular function as described herein. Such surfaces can be produced in large quantities in e.g. polymers like polystyrene, different types of polycaprolactones, Poly(methyl methacrylate, silicones including poly(dimethylsiloxane), poly(hydroxyethyl methacrylate), poly(ethyl methacrylate), poly(D,L-lactide-co-glycolide), polyethylene, polycarbonate, polyvinyl alcohols, hyaluronic acid-based polymers, poly(ethylene oxide), poly(butylene terephthalate), methacryloyloxyethyl phosphorylcholine, mr-I T85, mr-I 7030, poly(bis(trifluoroethoxy)phosphazenes, natural polymers including modified poly(saccharide)s, e.g., starch, cellulose, and chitosan, and mixtures and copolymers of the above mentioned, e.g. from a suitable stamp or blueprint by hot embossing or by injection molding, or by any other suitable process known in the art and/or as described herein. The surface 304 may be the upper surface of the bottom 303 or an upper surface of a separate element, e.g. a disk, placed on top of the bottom 303 of the dish. For example, such tissue culture dishes/flasks or any surface with structures selected to support the growth of embryonic stem cells in an undifferentiated state may be used to grow large quantities of embryonic stem cells that in a later development may be induced to differentiate into a desired cell type. Once differentiated into a specific cell type these specific cell types may be used for drug screening and/or cell replacement therapy.

The surface 304 may also be provided in the form of a separate tissue culture plastic that has been modified to display the selected structures or blueprint of these structures on the surface. For example, the separate tissue culture plastic may be removably inserted in the culture dish 301. In this respect the term tissue culture plastic is intended to include any polymers/metal coatings/material that can be used to produce a surface that can be used for growth of cells in vitro in cell culture.

### Examples

### Example 1

### Manufacture of a 4 inch BSSA wafer comprising 60 tester areas

A single-sided polished silicon wafer (4 inch) with a thickness of 525±25 µm provided a substratum for the manufacture of a biocompatible material. The wafer was an n-type wafer with a resistivity of 1-20 ohm cm. A micrometer-sized pattern was printed onto the polished side of the silicon wafer by standard photolithography and reactive ion etching in a SF₆/0₂ discharge according to the following protocol:
1. The wafers were pre-etched with buffered hydrofluoric acid (BHF, BHF is a solution of concentrated HF (49%), water, and a buffering salt, NH₄F, in about the ratio 1:6:4) for 30 seconds and then dried under N₂ flow, and
2. the wafer was then spin-coated with a 1.5 µm thick layer of photoresist AZ5214 , Hoechst Celanese Corporation, NJ, US (the chemical composition can be found at the Material Safety Data Sheet (MSDS) supplied by Hoechst Celanese Corporation).and pre-baked at around 90°C for 120 seconds, and
3. the photoresist-coated wafer was exposed to UV light for 5 seconds in an EVC aligner, model AL6-2 , through a suitable mask, allowed to develop for 50-60 seconds and then post-baked for 1 minute at 120°C, and
4. the photoresist-coated wafer was then patterned by briefly etching with BHF for approximately 30 sec., and then subjected to Reactive Ion Etching (RIE) at a rate of approximately 0.30µm/minute, and the resist was stripped with acetone followed by RCA cleaning. The RCA cleaning procedure has three major steps used sequentially: Removal of insoluble organic contaminants with a 5:1:1 H₂O:H₂O₂:NH₄OH solution (SC1). Removal of a thin silicon dioxide layer where metallic contaminants may have accumulated as a result of (I), using a diluted 50:1 H₂O:HF solution. Removal of ionic and heavy metal atomic contaminants using a solution of 6:1:1 H₂O:H₂O₂: HCl (SC2).
5. The patterned wafer was then passivated by dry oxidation with a 20 nm SiO₂ layer, thermally grown at 1000°C for 15 minutes.
6. A 250 nm tantalum layer was deposited onto the surface of the patterned wafer by sputter deposition.

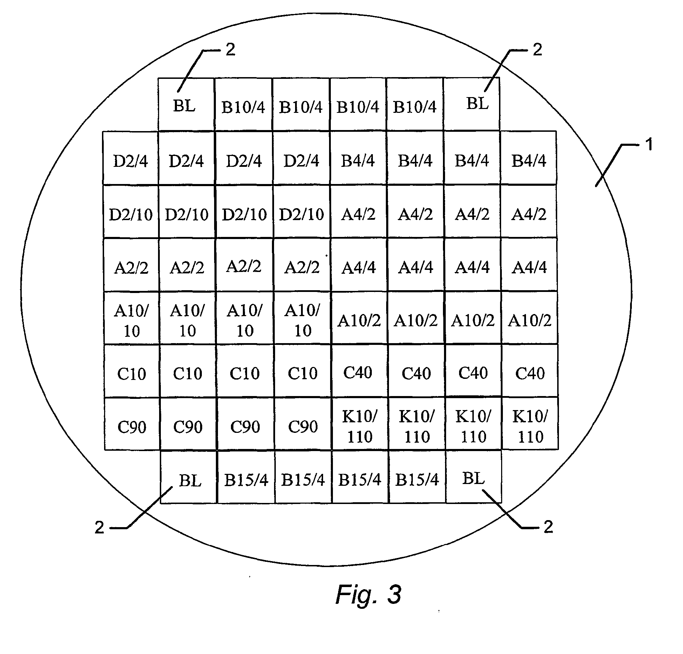

Fig. 3 shows a top view of one of the prepared wafers. The wafer was prepared comprising 60 tester areas, each with a dimension of 10mmx10mm, wherein each area has a specific lateral topography prepared according to Example 1. Each wafer includes four control tester areas 2 having a planar surface, and 4 replicates of each of 14 different lateral topologies. A series of wafers were produced according to these defined parameters, wherein the depth of the lateral topography was defined as either 0.07 µm, 0.25 µm, 0.60 µm, 1.20 µm, 1.60 µm.

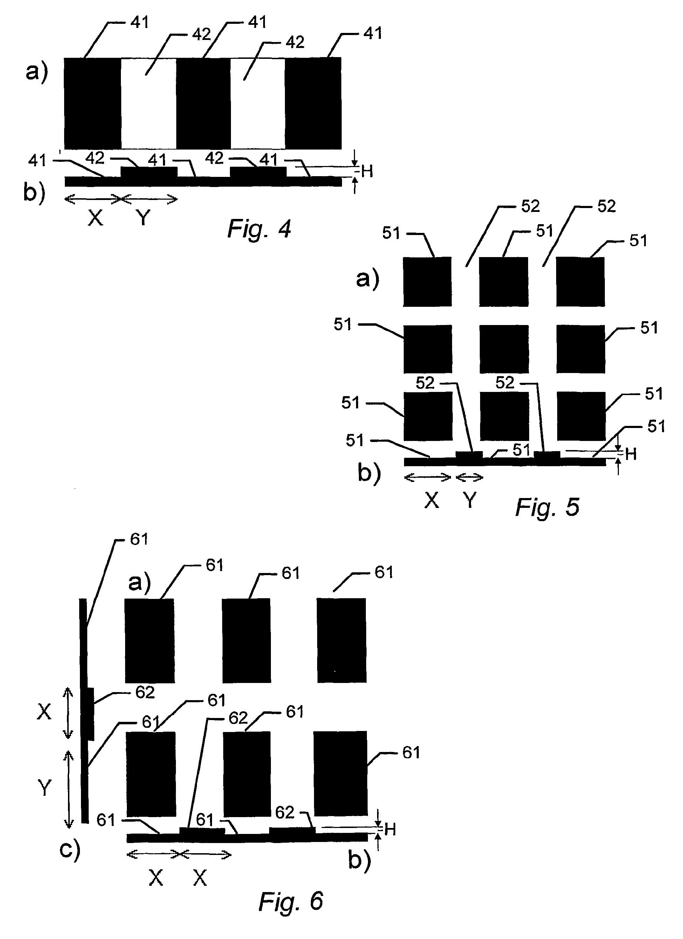

Each specific lateral structure is repeated 4 times. In fig. 3, each tester area is labelled to indicate its topographical surface structure, where the labels indicate the following structures:
- "BL": No structure, i.e. a substantially flat surface.
- "AX/Y": Line structures as shown in fig. 4. The structure includes trenches 41 of width X (in µm) and ridges 42 of width Y (in µm). Hence, the line structure of fig. 4 has micrometer scale features along one dimension only. In Example 1, the areas A2/2 include a line structures with trenches of width 2 µm and ridges of width 2µm, the areas A4/4 include line structures with trenches of width 4 µm and ridges of width 4µm, the areas A10/10 include line structures with trenches of width 10 µm and ridges of width 10µm, the areas A4/2 include line structures with trenches of width 4 µm and ridges of width 2µm, and the areas A10/2 include line structures with trenches of width 10 µm and ridges of width 2µm.
- "BX/Y": A square-hole structure as shown in Fig. 5, The structure includes square holes/recesses 51 with dimension X(in µm)×X(in µm) and a pitch distance of X+Y, i.e. the net of ridges 52 have a width of Y. Hence, the structure of fig. 5 has micrometer scale features in both dimensions within the plane of the surface of the tester area. In example 1, the areas B4/4 include square holes with dimension 4µm×4µm and pitch distance 8 µm, the areas B10/4 include square holes with dimension 10µm×10µm and pitch distance 14 µm, and the areas B15/4 include square holes with dimension 15µm×15µm and pitch distance 19 µm.
- "KX/Y": A structure comprising rectangular holes/recesses separated by ridges as shown in Fig. 6. The structure includes rectangular holes 61 of dimension X (in µm)×Y (in µm) separated with ridges 62 of width X (in µm). Hence, the areas K10/110 include rectangular holes with dimension 10µm×110µm separated with ridges of width 10µm.
- "DX/Y". A structure comprising protrusions/pillars as shown in Fig. 7. The structure comprises protrusions/pillars 71 with a square cross section of dimension X(in µm)×X(in µm) and a pitch distance of X+Y. Hence the areas D2/4 include a square-pillar structure with pillar dimensions 2 µm×2 µm and a pitch distance of 6 µm, and the areas D2/10 include a square-pillar structure with pillar dimensions 2 µm×2 µm and a pitch distance of 12 µm.
- "CX". A square-hole/pillar structure as shown in fig. 8. The structure has the appearance of a chess board with holes 81 and protrusions/pillars 82, both having the shape of squares with dimension X(in µm)×X(in µm). Hence, the areas C10 include a square-hole/pillar structure with dimension 10µm×10 µm of both holes and pillars, the areas C40 include a square-hole/pillar structure with dimension 40µm×40 µm of both holes and pillars, and the areas C90 include a square-hole/pillar structure with dimension 90µm×90 µm of both holes and pillars.

It is understood that the preparation method described above may also be applied to wafers with other forms and sizes of tester areas as well as other types of structures. The same production process may be used for a variety of different wafers, where the layout of the tester areas and the particular surface structures are determined by the mask through which the wafer is exposed.

### Example 2

### Screening a BSSA wafer identifies a biocompatible material for mineralisation of murine osteoblastic cells:

A number of wafers were produced as described in connection with example 1. Each wafer was placed in a P15 dish (NUNC, Biotech line) and washed with 70% ethanol and then PBS (6.8 g NaCl, 0.43 g KH₂PO₄, 0.978 g Na₂HPO₄*2H₂O in 1 liter double distilled water -pH 7.4). The wafer was seeded with cells of a MC3T3-E1 murine osteoblastic cell line (Sudo, H et al. 1983, J Cell Biol 96(1):191-98)*,* at a concentration of 20,000 cells/cm². The cells were cultured for 4 days in plain medium (alpha-minimal essential medium [α-MEM], 10% fetal calf serum [FCS], 100U/ml penicillin, and 100 microgram/ml streptomycin (supplied by Gibco, Invitrogen). The cells were maintained in a humidified incubator (5% CO₂/95% air atmosphere at 37°C), and subsequently 284µM ascorbic acid (Wako Chemicals, DE) and 10mM β-glycerophosphate (Sigma-Aldrich, DK) were included in the growth medium. The cells were cultured for 3 weeks, with a change of growth medium twice a week.
a) *In vitro* mineralisation. After 3 weeks culture, the wafers from each cell culture dish were tested for mineralization by washing the wafers with PBS and fixing the cells on the wafer with 70% ethanol for 1 hr at -20°C. The cells were then rinsed in double-distilled H₂O and then stained with 40 mM Alizarin Red S adjusted to pH 4.2 (Sigma-Aldrich, DK) for 10 minutes at room temperature (about 20-25°C). The wafers were post-rinsed with H₂O and incubated in PBS for 15 minutes to reduce non-specific staining.
b) Alizarin Red, that binds to calcium, stained all tester areas weakly, while the test areas having the microstructure D2/4 were strongly stained, as confirmed by cell growth on wafers performed in several independent experiments. D2/4 has a two-dimensional periodic structure of square pillars of dimension 2 µm x 2 µm and pitch distance of 6 µm (as described in connection with fig. 7).
c) A comparison of mineralisation on wafers with different depths of the lateral topography, revealed significant vertical dimension dependence for the mineralization process during cell culture. Enhanced mineralization on the test area D2/4, by comparison to the other test areas, was only detected when the vertical dimension of the lateral topography of D2/4 was greater than 0.6µm. Tester areas with a vertical dimension of 1.2 µm and 1.6 µm showed mineralization.

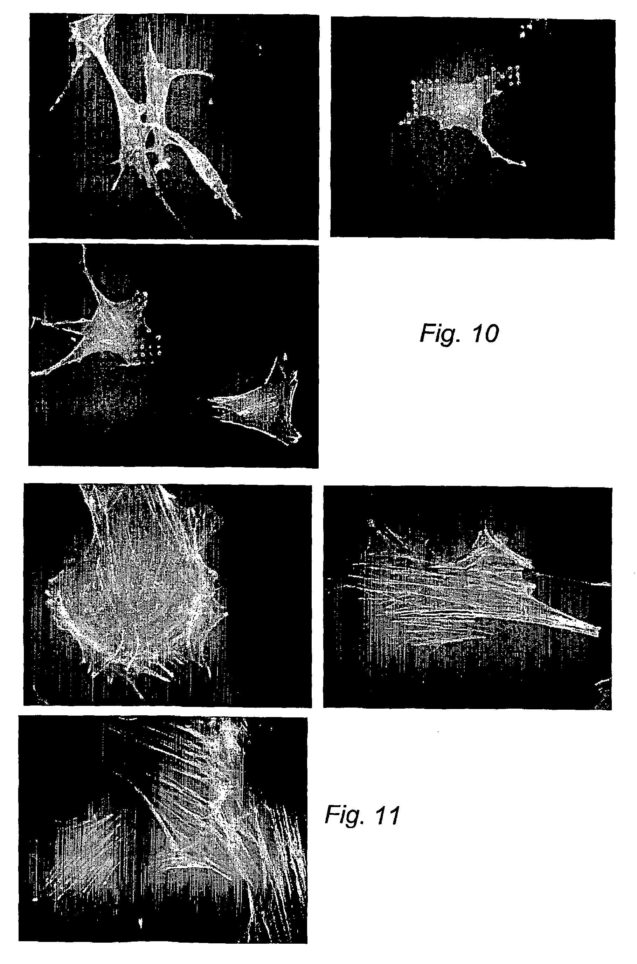

Figs. 9-11 show screening results of Osteogenic MC3T3 cells on tester areas of a BSSA wafer: Fig. 9a shows MC3T3 cells on glass, and Fig. 9b shows MC3T3 cells on a reference surface of a BSSA wafer. Fig. 10 shows MC3T3 cells on a tester area having a "D2/4" surface structure (H=1600nm). Fig. 11 shows MC3T3 cells on a tester area having a "D2/10" surface structure (1600nm). The MC3T3 cells shown in figs. 9-11 were fixed and stained with Rhodamin labeled Phalloidine, 48 hrs after seeding the cells. Phalloidine labels the actin filaments inside the cell. The cytoskeleton of the cells is seen to be differently affected by the surface topography. For the reference structure both on tantalum as on glass long actin fibers are seen to span the whole diameter of the cell. A similar pattern is observed for the non-mineralizing structure D2/10 though single dots of the structure are occasionally enhanced by the actin fibers. For the mineralizing structure D2/4 the pattern is clearly different. The cell shape is affected by the pattern on the surface as if the cell connects from dot to dot and the structure is highly observed by the fact that the actin skeleton seems to wrap around the single dots.

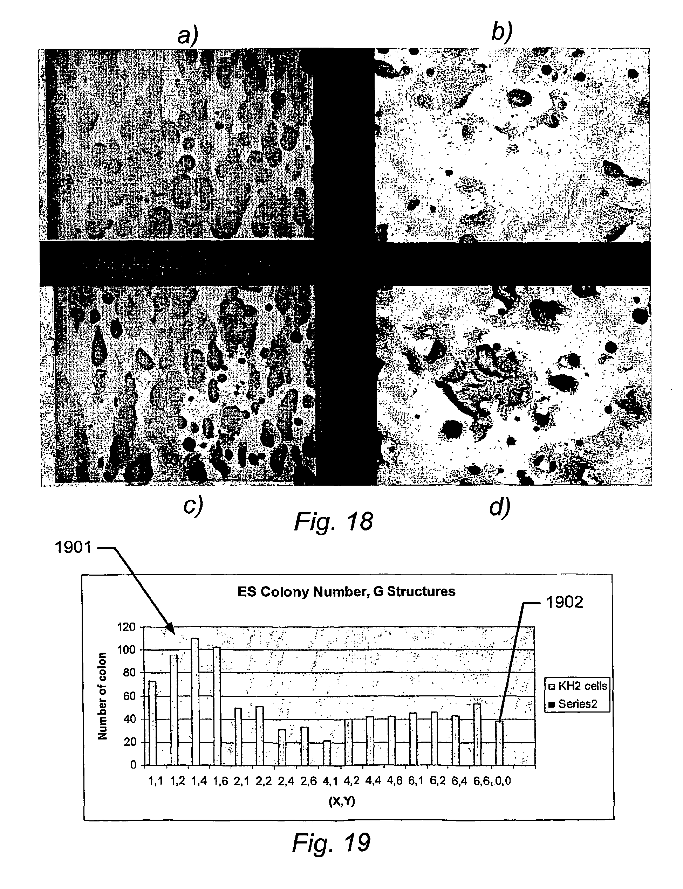

Contact points between the cells and the implant surface are the focal adhesion points where cellular proteins, like the integrins, make contact to peptide sequences as RGD in the extracellular matrix proteins deposited onto the surface. The intracellular domains of the integrins are associated with actin components of the cytoskeleton as well as proteins like vinculin, paxillin and focal adhesion kinase (FAK). These proteins mediate different cellular responses through different signaling pathways, and as such are expected to influence mineralization processes mediated by the cell. Another route where the intracellular distribution of the actin skeleton is expected to influence mineralization is through deposition of the extracellular matrix. The actin skeleton is known to determine the extracellular matrix proteins like fibronectin. Fibronectin is observed to be deposited along the actin fibers (see e.g. Molecular Biology of the Cell, Fourth Edition, by Bruce Alberts, Alexander Johnson, Julian Lewis, Martin Raff, Keith Roberts, and Peter Walter, fig. 19-54). Thus, it is likely that other extracellular matrix proteins e.g. collagen type I are affected by the actin skeleton. Collagen type I has been correlated to a high extent with the mineralization process of bone and osteoblasts. It is thought that the grooves within the collagen fibrils act as nucleation sites for the initial seeds of hydroxyapatite crystals. We expect that changes within the topography of the implant surface will lead to changes within the cytoskeleton and hence, affect the above mentioned processes.

### Example 3

### Identification of biocompatible surfaces for mineralization of osteogenic MC3T3 cells:

A BSSA wafer comprising tester squares having topographical structures selected from the structures identified in fig. 12 or structures modified from the structures identified in fig 12, was prepared.

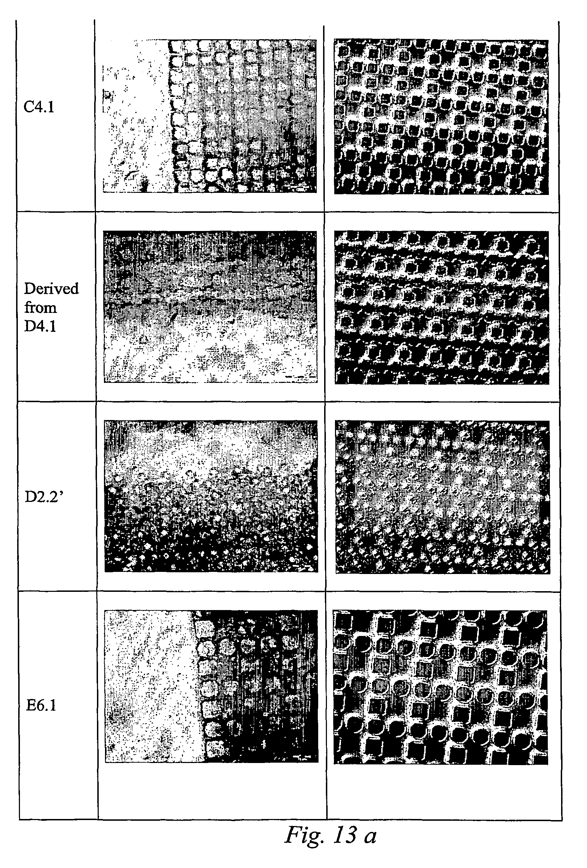

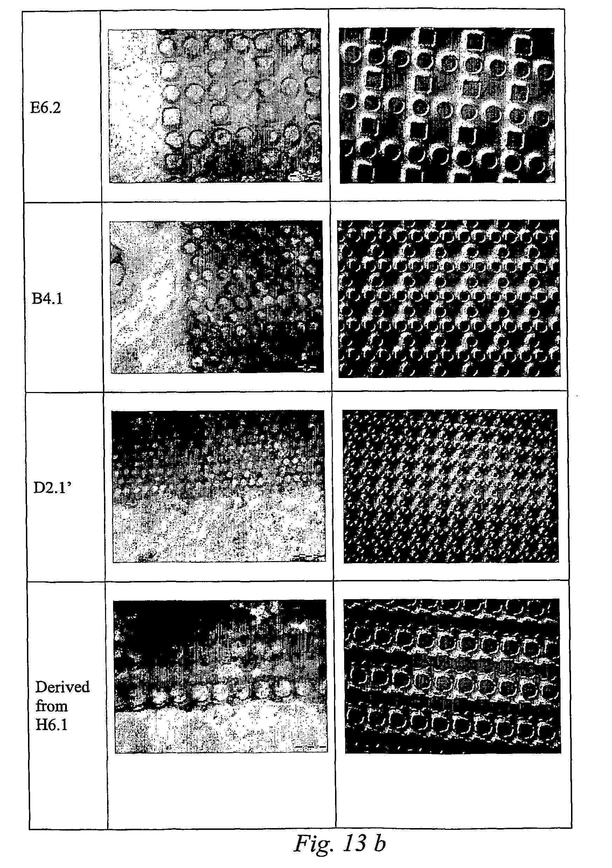

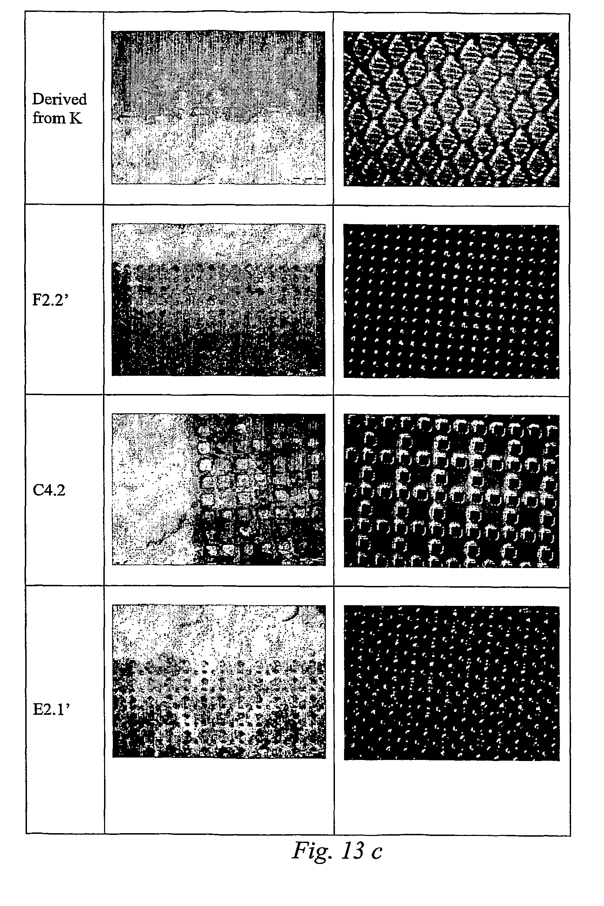

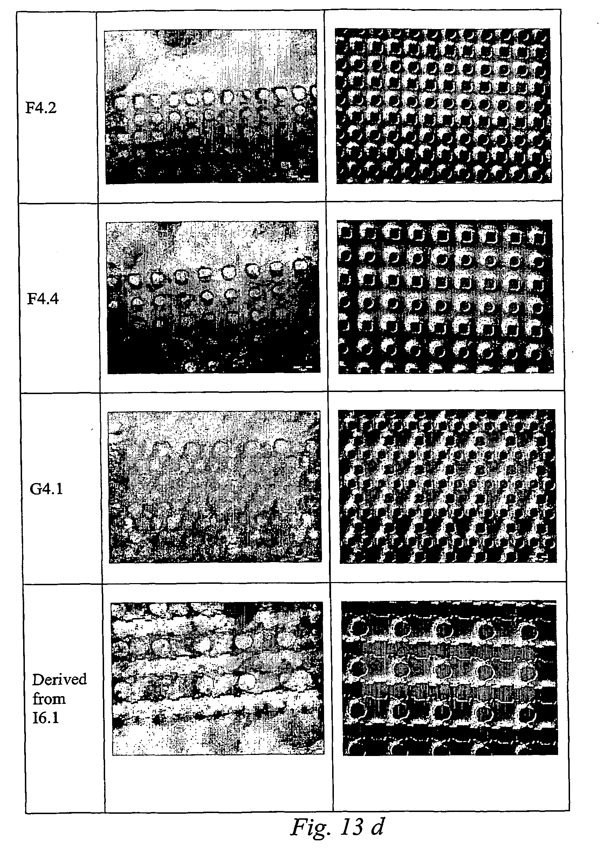

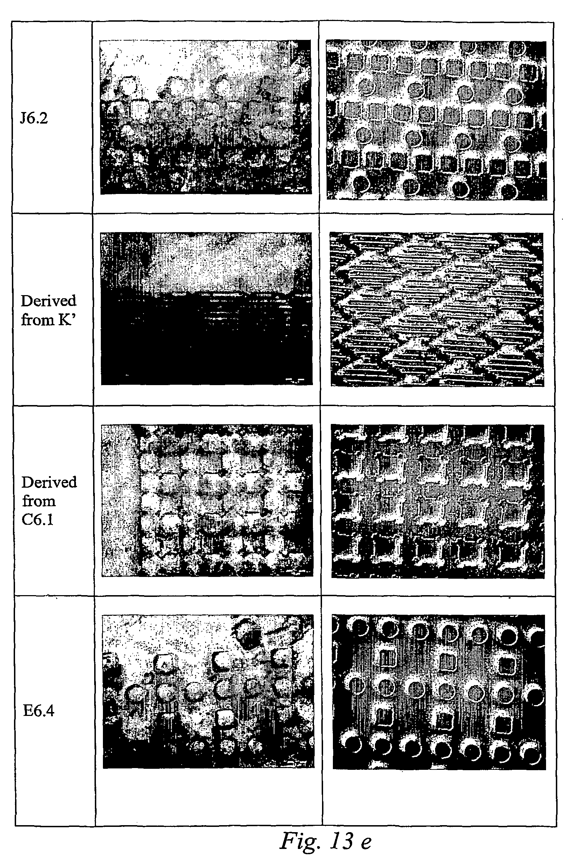

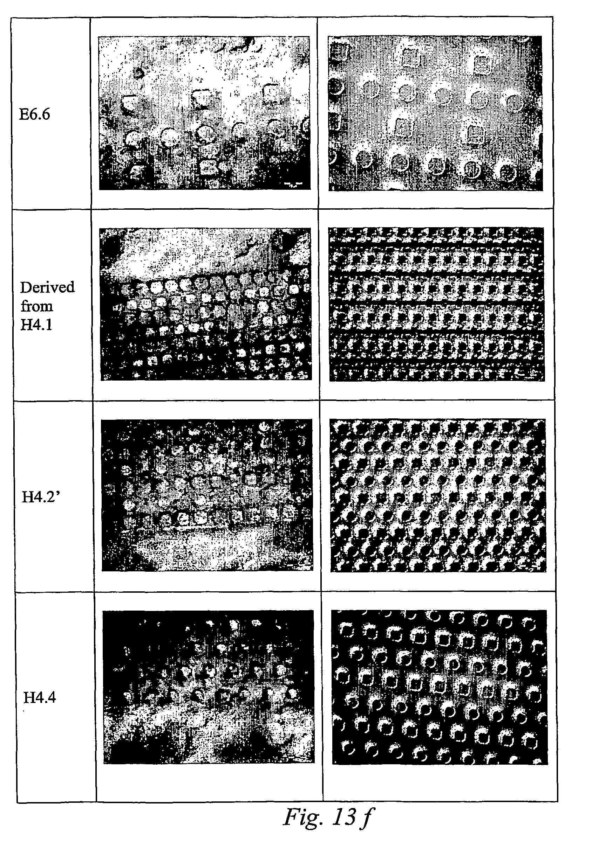

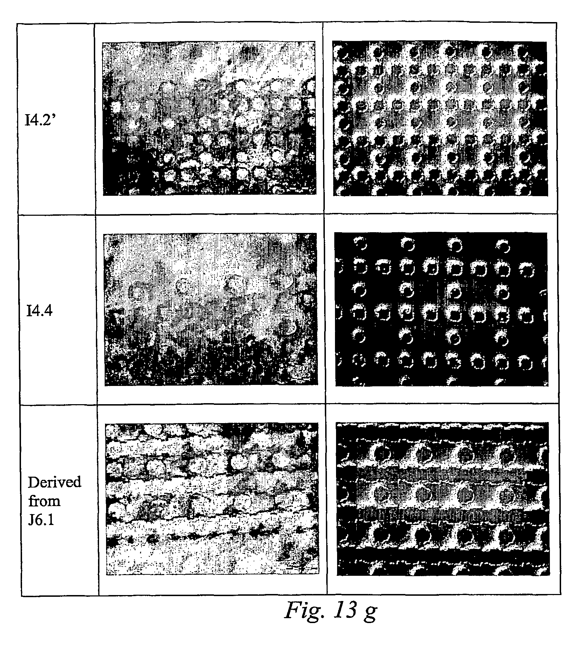

A wafer, comprising tester areas having the topographical structures shown in fig. 12 a-k, or structural modifications thereof, was seeded with MC3T3 cells, cultured, and subsequently stained for mineralization employing the alizarin red assay, as described in Example 2, and the level of mineralization was scored based on visual inspection. Images of surface structures found to be particularly favorable for mineralization, and thus shown to be biocompatible for bone-forming cells, are shown in fig. 13. Each of figs. 13a-g shows a table, where each row corresponds to one of the identified structures. The first (left-most) column comprises identification codes for the respective structures, the second column shows respective parts of a tester area of a wafer with cells stained for mineralization with Alizarin Red, and the third (right-most) row shows the corresponding tester areas without cells. In the images showing the areas with cells, edges of the respective tester squares are shown as to allow comparison with a non-structured surface. The identified surface structures were (in the following the structures are identified by the codes according to fig. 13):

**B4.1**. A structure comprising circles alone as shown in figs. 12b and 13b, second row. B4.1 includes a circular-pillar structure with a diameter of X=4µm and a pitch distance of X+Y=5µm in each second row (i.e. Y=1µm). The remaining rows include a circular-pillar structure with a diameter of X=4µm and a pitch distance of 2(X+Y)=10µm. The pitch distance between the rows is 5µm. The pillars of the rows with pitch distance 10µm are aligned with the center of the gaps between the pillars of the rows with pitch distance of 5µm.

**C4.1.** A structure comprising squares alone as shown in figs. 12c and 13a, first row. C4.1 includes a square-pillar structure with pillar dimensions 4µm x 4µm (i.e. X=4µm) and a pitch distance of X+Y=5µm in each second row (i.e. Y=1µm). The remaining rows include a square-pillar structure with pillar dimensions 4µm x 4µm and a pitch distance of 10µm. The pitch distance between the rows is 5µm. The squares of all rows are aligned in columns, i.e. squares of adjacent rows are placed above each other.

**C4.2.** A structure comprising squares alone as shown in figs. 12c and 13c, third row. C4.2 includes a square-pillar structure with pillar dimensions 4µm x 4µm (i.e. X=4µm) and a pitch distance of X+Y=6µm in each second row (i.e. Y=2µm). The remaining rows include a square-pillar structure with pillar dimensions 4µm x 4µm and a pitch distance of 12µm. The pitch distance between the rows is 6µm. The squares of all rows are aligned in columns, i.e. squares of adjacent rows are placed above each other.

**E6.1.** A structure comprising squares and circles in the relative amount of 1:2 as shown in fig. 12e and 13a, fourth row. E6.1 includes a square-pillar structure with pillar dimensions 6µm x 6µm and a pitch distance of 14µm in each second row. The other second row includes a circular-pillar structure with a diameter of 6µm and a pitch distance of 7µm. The pitch distance between the rows is 7µm (i.e. X=6µm, Y=1µm). The pillars within the rows are placed above each other.

**E6.2.** A structure comprising squares and circles in the relative amount of 1:2 as shown in figs. 12e and 13b, first row. E6.2 includes a square-pillar structure with pillar dimensions 6µm x 6µm and a pitch distance of 16µm in each second row. The other second row includes a circular-pillar structure with a diameter of 6µm and a pitch distance of 8µm. The pitch distance between the rows is 8µm (i.e. X=6µm, Y=2µm). The pillars within the rows are placed above each other.

**E6.4.** A structure comprising squares and circles in the relative amount of 1:2 as shown in figs. 12e and 13e, fourth row. E6.4 includes a square-pillar structure with pillar dimensions 6µm x 6µm and a pitch distance of 20µm in each second row. The other second row includes a circular-pillar structure with pillar diameters of 6µm and a pitch distance of 10µm. The pitch distance between the rows is 10µm (i.e. X=6µm, Y=4µm). The pillars within the rows are placed above each other.

**E6.6.** A structure comprising squares and circles in the relative amount of 1:2 as shown in figs. 12e and 13f, first row. E6.6 includes a square-pillar structure with pillar dimensions 6µm x 6µm and a pitch distance of 24µm in each second row. The other second row includes a circular-pillar structure with pillar diameters of 6µm and a pitch distance of 12µm. The pitch distance between the rows is 12µm (i.e. X=6µm, Y=6µm). The pillars within the rows are placed above each other.

**F4.2.** A structure comprising squares and circles in the relative amount of 1:1 as shown in figs. 12f and 13d, first row. F4.2 includes a square-pillar structure with pillar dimensions 4µm x 4µm and a pitch distance of 6µm in each second row. The other second row includes a circular-pillar structure with pillar diameters of 4µm and a pitch distance of 6µm. The pitch distance between the rows is 6µm (i.e. X=4µm, Y=2µm). The pillars within the rows are placed above each other.

**F4.4.** A structure comprising squares and circles in the relative amount of 1:1 as shown in figs. 12f and 13d, second row. F4.4 includes a square-pillar structure with pillar dimensions 4µm x 4µm and a pitch distance of 8µm in each second row. The other second row includes a circular-pillar structure with pillar diameters of 4µm and a pitch distance of 8µm. The pitch distance between the rows is 8µm (i.e. X=4µm, Y=4µm). The pillars within the rows are placed above each other.

**G4.1**. A structure comprising squares and circles in the relative amount of 1:2 as shown in figs. 12g and 13d, third row. G4.1 includes a square-pillar structure with modified/roughened edges and pillar dimensions 4µm x 4µm and a pitch distance of 10µm in each second row. The other second row includes a circular-pillar structure with modified/roughened edges and pillar diameters of 4µm and a pitch distance of 5µm. The pitch distance between the rows is 5µm (i.e. X=4µm, Y=1µm). The squares within the rows are placed between each other, i.e. aligned with the gaps between the circular pillars of the adjacent rows.

**H4.4.** A structure derived from the structure of fig. 12h comprising squares with modified/roughened edges and circles in the relative amount of 1:1 as shown in fig. 13f, fourth row. H4.4 includes a square-pillar structure with pillar dimensions 4µm x 4µm and a pitch distance of 8µm in each second row. The other second row includes a circular-pillar structure with pillar diameters of 4µm and a pitch distance of 8µm (i.e. X=4µm, Y=4µm). The pitch distance between the rows is 8µm. The pillars within the rows are placed between each other. In this example the squares with roughened edges have a generally square cross-section, where the edges of the square are formed by curved lines rather than straight lines, thus resulting in a square with ruffled edges.

**I4.4.** A structure derived from the structure of fig. 12i comprising squares with roughened edges as above and with circles in the relative amount of 2:1 as shown in fig. 13g, second row. 14.4 includes a square-pillar structure with pillar dimensions 4µm x 4µ.m and a pitch distance of 8µm in each second row. The other second row includes a circular-pillar structure with pillar diameters of 4µm and a pitch distance of 16µm. The pitch distance between the rows is 8µm. The pillars within the rows are placed above each other.

**J6.2.** A structure comprising squares and circles in the relative amount of 2:1 as shown in figs. 12j and 13e, first row. J6.2 includes a square-pillar structure with pillar dimensions 6µm x 6µm and a pitch distance of 8µm in each second row. The other second row includes a circular-pillar structure with pillar diameters of 6µm and a pitch distance of 16µm. The pitch distance between the rows is 8µm (i.e. X=6µm, Y=2µm). The pillars within the rows are placed between each other.

**D2.1'** (modified). A structure as shown in fig. 13b, third row, that is derived from the structure of fig. 12d comprising squares alone but with a 45° rotation of the squares as compared to the structure shown fig. 12d. D2.1' includes a square-pillar structure where the squares have curved/rounded edges and with pillar dimensions 2µm x 2µm and a pitch distance of 3µm in each second row. The other second row includes a square-pillar structure with pillar dimensions 2µm x 2µm and a pitch distance of 6µm (i.e. X=2µm, Y=1µm). The pitch distance between the rows is 3µm. The rows are shifted to place the squares between each other.

**D2.2'** (modified). A structure as shown in fig. 13a, third row, that is derived from the structure of fig. 12d comprising squares alone but with a 45° rotation of the squares as compared to the shown fig. 12d. D2.2' includes a square-pillar structure with roughened borders and pillar dimensions 2µm x 2µm and a pitch distance of 4µm in each second row. The other second row includes a square-pillar structure with pillar dimensions 2µm x 2µm and a pitch distance of 8µm. The pitch distance between the rows is 4µm (i.e. X=2µm, Y=2µm). The rows are shifted to place the squares between each other.

**E2.1'** (modified). A structure as shown in fig. 13c, fourth row, that is derived from the structure of fig. 12e comprising squares and circles in the relative amount of 1:2 but with a 45° rotation of the squares as compared to the shown fig. 12e. E2.1' includes a square-pillar structure with pillar dimensions 2µm x 2µm and a pitch distance of 6µm in each second row. The other second row includes a circular-pillar structure with pillar diameters of 2µm and a pitch distance of 3µm. The pitch distance between the rows is 3µm (i.e. X=2µm, Y=1 µm). The rows are shifted to place the pillars above each other.

**F2.2'** (modified). A structure as shown in fig. 13c, second row, that is derived from the structure of fig. 12f, comprising squares and circles in the relative amount of 1:1 but with a 45° rotation of the squares as compared to the shown fig. 12f. F2.2' includes a square-pillar structure with pillar dimensions 2µm x 2µm and a pitch distance of 4µm in each second row. The other second row includes a circular-pillar structure with pillar diameters of 2µm and a pitch distance of 4µm. The pitch distance between the rows is 4µm (i.e. X=2µm, Y=2µm). The rows are shifted to place the pillars above each other.

**H4.2'** (modified). A structure as shown in fig. 13f, third row, that is derived from the structure of fig. 12h, comprising squares and circles in the relative amount of 1:1 but with the squares modified as to have a flower-like shape as compared to the illustrated in fig. 12h. H4.2' includes a flower-pillar structure with pillar dimensions 4µm x 4µm and a pitch distance of 6µm in each second row. The other second row includes a circular-pillar structure with pillar diameters of 4µm and a pitch distance of 6µm. The pitch distance between the rows is 6µm (i.e. X=4µm, Y=2µm). The rows are shifted as to place the pillars between each other.

**I4.2**' (modified). A structure as shown in fig. 13g, first row, that is derived from the structure of fig. 12i, comprising squares and circles in the relative amount of 2:1 but with the squares modified as to have a flower-like shape as compared to the illustrated in fig. 12i. I4.2' includes a flower-pillar structure with pillar dimensions 4µm x 4µm and a pitch distance of 6µm in each second row. The other second row includes a circular-pillar structure with pillar diameters of 4µm and a pitch distance of 12µm. The pitch distance between the rows is 6µm (i.e. X=4µpm, Y=2µm). The rows are shifted as to place the pillars above each other.

**Derived from C6.1**. A structure as shown in fig. 13e, third row that is derived from the structure of fig. 12c, comprising horizontal and vertical ridges originally derived from the structure as shown in fig. 12c. The structure includes crossing lines with diameters ranging from 3-6µm and a pitch distance of maximum 144µm (i.e. X=6µm, Y=1µm). The ridges have side faces include lateral protrusions resulting in multiple edges/angles.

**Derived from D4.1**. A structure as shown in fig. 13a, second row, comprising squares and lines originally derived from the structure shown in fig. 12d. The structure includes a square-pillar structure with pillar dimensions 4µm x 4µm and a pitch distance of 10µm in each second row. The squares appear with cut corners as shown in fig. 13a, second row. The other second row includes a line/ridge structure with multiple angles and a width ranging from 1-4µm. The pitch distance between the rows is 5µm (i.e. X=4µm, Y=1 µm).

**Derived from H4.1.** A structure as shown in fig. 13f, second row, comprising circles and lines/ridges originally derived from the structure as shown in fig. 12h. The structure includes a circular-pillar structure with pillar diameters of 4µm and a pitch distance of 5µm in each second row. The circles appear with modified/roughened edges as shown in fig. 13f, second row. The other second row includes a line structure with multiple angles and a diameter ranging from 1-4µm. The pitch distance between the rows is 5µm (i.e. X=4µm, Y=1 µm).

**Derived from H6.1.** A structure as shown in fig. 13b, fourth row, comprising circles and lines originally derived from the H structure shown in fig. 12h. The structure includes a circular-pillar structure with pillar diameters of 6µm and a pitch distance of 7µm in each second row. The circles appear with roughened borders as shown in fig. 13b, fourth row. The other second row includes a line structure with multiple angles and a diameter ranging from 3-6µm. The pitch distance between the rows is 7µm (i.e. X=6µm, Y=1µm). **Derived from I6.1.** A structure as shown in fig. 13d, fourth row, comprising circles and lines originally derived from the structure shown in fig. 12i. The structure includes a circular-pillar structure with pillar diameters of 6µm and a pitch distance of 14µm in each second row. The circles appear with rough borders as shown in fig. 13d, fourth row. The other second row includes a line structure with multiple angles and a diameter ranging from 4-6µm. The pitch distance between the rows is 7µm (i.e. X=6µm, Y=1µm).

**Derived from J6.1.** A structure as shown in fig. 13g, third row, comprising circles and lines originally derived from the structure shown in fig. 12j. The structure includes a circular-pillar structure with pillar diameters of 6µm and a pitch distance of 14µm in each second row. The circles appear with rough edges as shown in fig. 13g, third row. The other second row includes a line structure with multiple angles and a diameter ranging from 4-6µm. The pitch distance between the rows is 7µm (i.e. X=6µm, Y=1µm).

**Derived from K.** A structure as shown in fig. 13c, first row, comprising groups of 6 lines/rectangles each, where each group of lines are arranged to form a rectangular shape. The structure is derived from the structure shown in fig. 12k. The rectangular shape includes lines with the dimensions x, 2x, 3x, 3x, 2x, x with linear dimensions of x∼1.8µm and a width of ∼1µm. The distance between the lines is ∼1µm. The rows are shifted as to place the rectangles between each other.

**Derived from K'**. A structure as shown in fig. 13e, second row, comprising groups of 7 lines/rectangles each, where each group of lines are arranged to form a rectangular shape. The structure is derived from the structure shown in fig. 12k. The rectangular shape includes lines with the dimensions x, 3x, 5x, 7x, 5x, 3x, x with linear dimensions of x∼2.8µm and a width of ∼1µm. The distance between the lines is ∼1µm. The rows are shifted to place the rectangles between each other.

In conclusion, on all of these data it can be seen that the staining intensity is highly increased by the given structures as compared with the reference structure (flat surface). Especially, the mineralization seems to be placed within angles and closely around the structures.

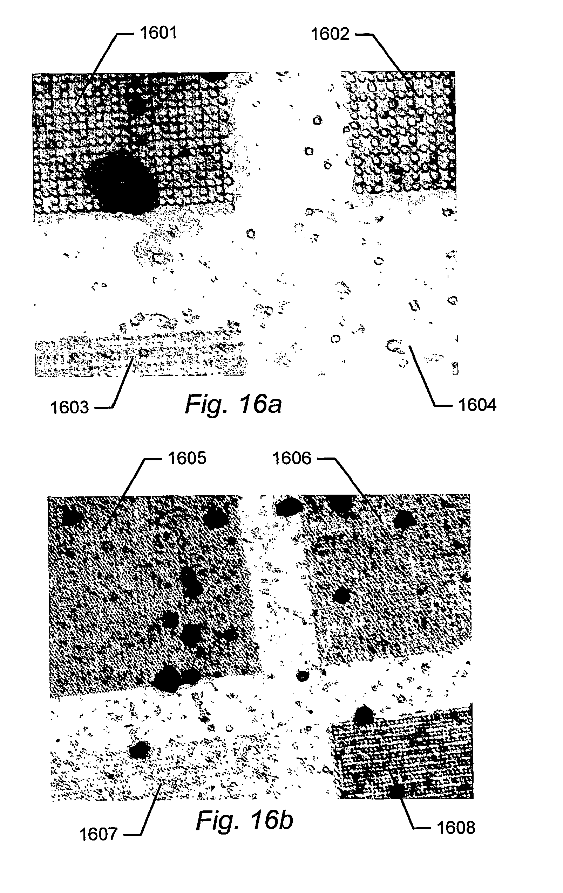

Fig. 16 shows two examples of views of a section of a BSSA wafer showing parts of four tester squares. A four inch wafer with tester squares of 4x4 mm was constructed. Figs. 16a-b each shows parts of four such tester squares. MC3T3 cells were seeded on the wafer and induced to mineralize as described for the 10 mm wafer of example 1 and 2. The wafer was subsequently stained for Ca using alizarin red as described for the 10 mm wafer. The surface structures of the tester squares were as follows: Tester square 1601: E6.1 (i.e. a structure as shown in fig. 12e with X=6, Y=1); tester square 1602: E6.2 (i.e. a structure as shown in fig. 12e with X=6, Y=2); tester square 1603: F2.4 (i.e. a structure as shown in fig. 12f with X=2, Y=4); tester square 1604: blanc (no structure); tester square 1605: G4.1 (i.e. a structure as shown in fig. 12g with X=4, Y=1); tester square 1606: G4.2 (i.e. a structure as shown in fig. 12g with X=4, Y=2); tester square 1607: H4.6 (i.e. a structure as shown in fig. 12h with X=4, Y=6); tester square 1608: H6.1 (i.e. a structure as shown in fig. 12h with X=6, Y=1).

These two examples clearly show the difference in mineralization ability of the different structures. First of all the staining for mineralization is seen to be delimited by the border of the structure illustrating the importance of the structure. Secondly, a difference in staining intensity is observed e.g. 1604 and 1607 are seen to be less potent for mineralization than e.g. 1601 and 1608.

### Example 4: Gene induction assay in combination with mineralization inducing genes

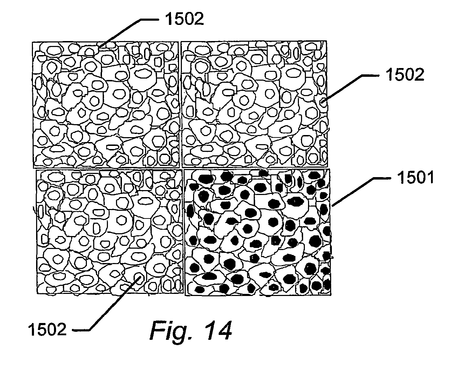

A gene induction assay as described below with reference to fig. 14 may be used in combination with mineralization inducing genes. This example of a gene induction assay uses primary cells from Knock-in mice. For live cells reporter systems using EGFP or other fluorescent protein expressing reporter systems can be used to construct reporter constructs to replace naturally occurring genes. After homologous recombination in ES cells knock-in mice are generated, e.g. as described in "Dmd(mdx-beta geo): a new allele for the mouse dystrophin gene" by K. Wertz and EM. Fuchtbauer, Dev Dyn. 1998 Jun;212(2):229-41. From these mice relevant primary cells are isolated. Cells from such mice express the EGFP construct when the targeted gene is induced. Fig 14 shows 4 tester areas of a BSSA wafer as an example of such a screening for an osteoinductive surface. The tester area 1501 an osteoinductive surface while tester areas 1502 comprise non osteoinductive surfaces. It is understood that a variety of other reporter systems can be used in this setup. One example is a Beta-galactosidase expressing Knock-in mice. A collection of more than 6000 different Knock-in mice exist using this expressing system as described in "A large-scale, gene-driven mutagenesis approach for the functional analysis of the mouse genome" by Hansen J, Floss T, Van Sloun P, Fuchtbauer EM, Vauti F, Arnold HH, Schnutgen F, Wurst W, von Melchner H, Ruiz P, Proc Natl Acad Sci U S A, 2003 Aug 19;100(17):9918-22. Epub 2003 Aug 6. ]

### Example 5: Ectopic bone formation

Ectopic bone formation may be analyzed as follows: 500,000 MC3T3-E1 cells per cm² are cultured on the 6 mmx6 mm biocompatible material structure D2/4, in plain medium including added 50 microgram/ml ascorbic acid and 10 mM beta-glycerophosphate. After one week in culture, the biocompatible material structures are transferred to the subcutaneous mouse model for ectopic bone formation. Two pouches are made subcutaneously on the dorsal surface of the mice. During surgery the animals are anesthetized with isofluorane and one structure is put into each pouch, which are closed using surgical sewing. The mice are left for 8 weeks before they are killed by cervical dislocation. The structures are removed, plastic embedded in poly-methylmethacrylate (PMMA), cut, and stained for bone detection (e.g. basic fuchsin/light green or Alizarin Red S).

### Example 6. Bone-forming assay in sheep

An *in vivo* sheep model as described below can be used to assay the ability of the biocompatible material to induce bone formation/ingrowth:

Samples of the relevant biocompatible material and controls, e.g. flat tantalum, are produced on the base of 6mmx6mm silicon wafer squares. The samples are named "active" and "control", respectively, and are used in pairs to reduce the variation of the results due to animal variations The samples are glued on sample holders, by a biocompatible glue (e.g. Loctite 431) creating an implant.

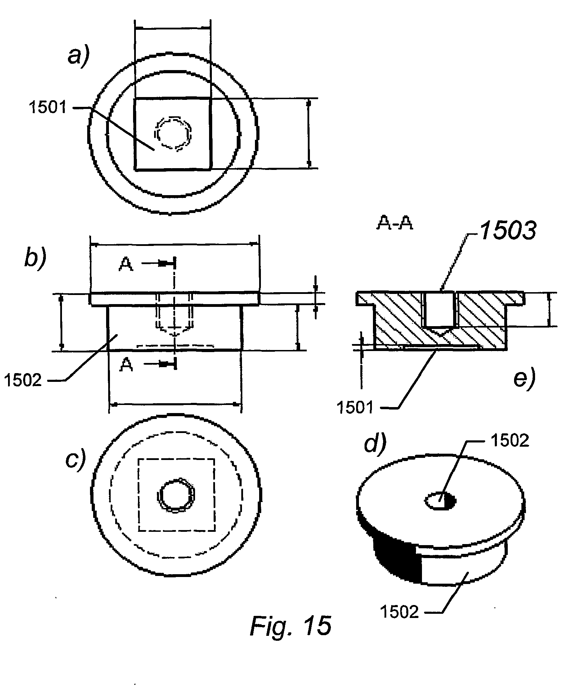

The sample holder for use in a bone-forming assay in sheep is illustrated in Figure 15. In particular, fig. 15a shows a bottom view of the sample holder, fig. 15b shows a side view of the sample holder, fig. 15c shows a top view of the sample holder, fig. 15d shows a perspective view of the sample holder, and fig. 15e shows a cross-sectional view of the sample holder. The sample holder comprises a square recess 1501 adapted to receive the sample whose biocompatible surface is to be exposed. The square recess is located in the bottom surface of a cylindrical member 1502. In the top surface the sample holder comprises a threaded hole 1503 facilitating placement and removal of the sample holder in/from the bone.

Before surgery the sheep are given 2.5 ml Rompun vet. and 2 ml. Atropin. After 20 min. the animals are anesthetized with 15 ml. Propofol. In each medial femoral condyl one hole is drilled with depth 6 mm and diameter 11 mm. This leaves 0.5 mm gap between the biocompatible surface and the bone for examination of the bone ingrowth. The implants are press-fitted into the hole and the cut is closed by surgical sewing. The sheep are left for four weeks after which they are sacrificed. The implants are removed and embedded in poly-methylmethacrylate (PMMA). The degree of bone ingrowth is initially examined by µCT-scanning followed by cutting and standard histological examination of bone volume and bone ingrowth towards the implant.

### Example 7

### Mouse embryonic stem (ES) cells

ES cells (KH2 cells) were seeded upon BSSA wafers and expanded for one passage. The cells were seeded at a density from 1.3-5x10⁶ cells/p10 Petri dish. Culturing conditions were 5% CO₂, 90% air humidity and 37°C. The cells were grown in DMEM supplemented with 15% FCS, 2mM glutamine, 50 U/ml penicillin, 50 µg/ml streptomycin, non-essential amino acids, 100 µM β-mercaptoethanol and nucleosides. Furthermore, the ES cell growth medium were supplemented with Leukemia Inhibitory Growth factor (LIF), 1000 U/ml, for maintaining the undifferentiated phenotype of the ES cells. The cells are stained for alkaline phosphatase (AP) activity (Blue). The level of AP is high in undifferentiated murine ES cells. Traditionally the ES cells are grown on a feeder layer of cells and passaged every second day. In this case the cells were passaged for the three days before fixation but without a feeder layer.

Fig. 17 shows experimental results that illustrate how selected structures increase the number of characteristic embryonic stem cell colonies in a cell culture as compared to a control structure. Fig. 17a shows a picture of stained ES on a structure D1.2 (as described in connection with figs. 12d), Fig. 17b shows a picture of stained ES on a control surface (no structure), while Fig. 17c shows a picture of stained ES on a structure H1.2 (as described in connection with figs. 12h).

The selected structures D1.2 and H1.2 increase the number of characteristic ES cell colonies as compared to the control surface (they appear rounded with a heavy staining due to a high AP activity).

Fig. 18 shows experimental results that illustrate how a selected structure resembling sharkskin directs differentiation as compared to a control structure. Figs. 18a and c show ES on the structure K2 (as described in connection with fig. 12k), while Figs. 18b and d show ES on a control surface (no structure). In Figs. 18 a and b, no LIF was added, while in Figs. 18 c and d, LIF was added. The cells that obtained LIF had LIF for one day succeeded by medium without LIF for two days. The Structure K2 thus directs differentiation as compared to the control structure. It can further be seen that the cell density on the structured surface is higher than on the unstructured surface.

Fig. 19 shows quantitative results illustrating how structures within selected size ranges increase the number of characteristic embryonic stem cell colonies in a cell culture as compared to a control structure. In particular, Fig. 19 shows quantitative results for a series of structures G*X. Y* (as described in connection with fig. 12g) for different values of X and Y. As can be seen from fig. 19, the structures with a diameter of one micrometer and a gap size between 1 to 6 micrometers, and more particularly between 2 to 6 micrometers (indicated by reference numeral 1901) enhance the number of ES colonies formed. The structure labeled 0,0 (reference number 1902) corresponds to the unstructured reference.

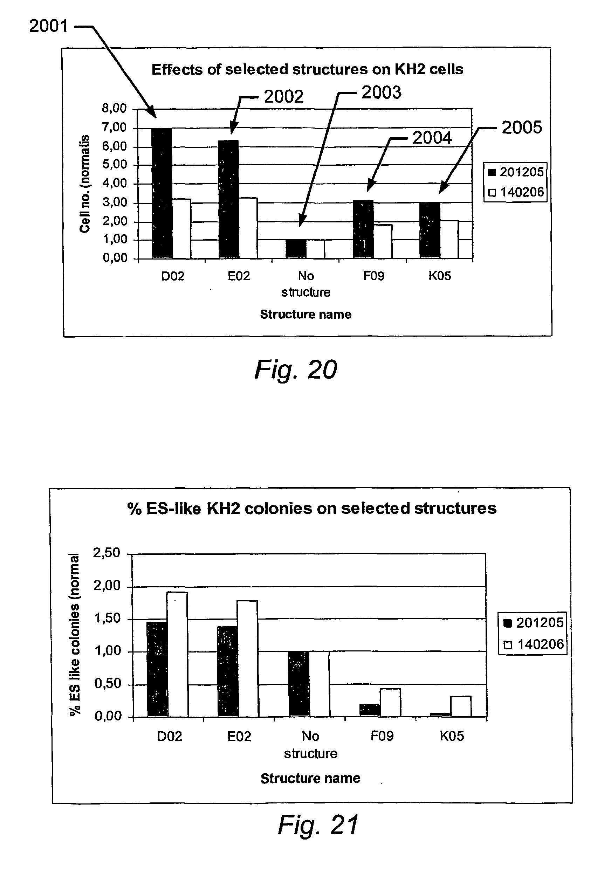

Fig. 20 shows quantitative results illustrating how some selected structures increase the number of characteristic embryonic stem cell colonies in a cell culture as compared to control structures. In particular, fig. 20 shows cell count results for ES on structures D1.2 (2001), E1.2 (2002), on an unstructured surface (2003), on structure F4.1 (2004), and on structure K5 (2005). Hence, a number of independent experiments show that some structures (D1.2, E1.2) enhance the formation of ES colonies as compared to the unstructured reference (2003) surface as well as compared to other structures (2004, 2005). The number of colonies has been normalized to the unstructured reference surface.

Fig. 21 shows quantitative results illustrating how some structures enhance the quality of the embryonic stem cell colonies with respect to the phenotypic appearance of the embryonic stem cell colonies (more rounded and smaller) while other structures guide the embryonic stem cells down the differentiation pathway. The data has been normalized to the control structure. The ES cells were grown with LIF for tree days on wafer without passaging.

In summary, the above results show that some structures support the growth of undifferentiated (feederdependent) murine ES cells without the inclusion of a layer of feeder cells. In particularly, structures D1.2, E1.2, H1.2 have been found to have this effect. Nevertheless, it has been found that generally structures with a feature diameter of about one micrometer and a minimum gap size between adjacent/nearest-neighbor features of between 2 to 6 micrometer have this effect. On the other hand, other types of structures, like the complex K structures, drive the ES cells down the differentiation pathway (e.g. K2).

### Example 8

### Neuronal differentiation of embryonic stem cells

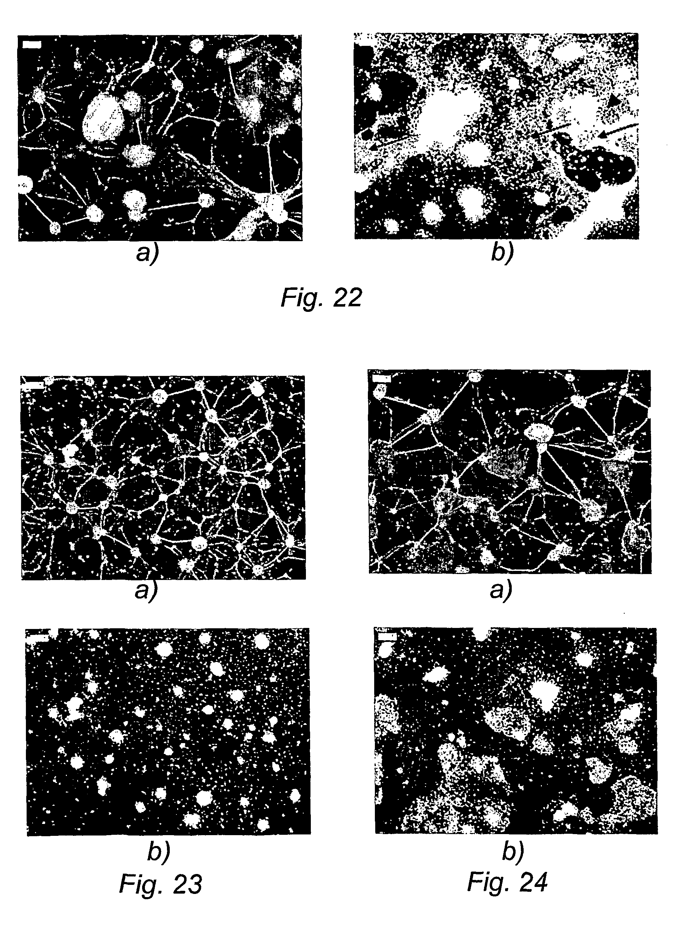

The murine embryonic stem (ES) cells, KH2, are differentiated into neurons using a protocol that induces differentiating toward glutamatergic neurons modified from (Bibel 2004, Nat. Neurosci. 7(9), p 1003): After expansion of the ES cells in the presence of Leukaemia Inhibitory Factor (LIF) the cells are transferred to non-adhered dishes in absence of LIF to induce the formation of embryonic bodies (EB). Retinoic acid is added after four days and the EBs continue to grow for four more days. EBs are then dissociated and single cells are transferred to a BSSA wafer, coated with poly-D-lysine, in N2 medium (DMEM:F12 (1:1) with N2 supplement). After two days, medium is changed to neurobasal medium with B27 supplement and cells are grown for further 3 to 14 days. Figs. 22-26 show pictures of cells allowed to differentiate for 14 days in B27 medium, fixed, and stained with antibodies against β-tubulin III (neuronal marker, red) (Figs. 22a, 23a, 24a, 25a, and 26a) and DAPI (which stain cell nuclei, blue) (Figs. 22b, 23b, 24b, 25b, and 26b), respectively.

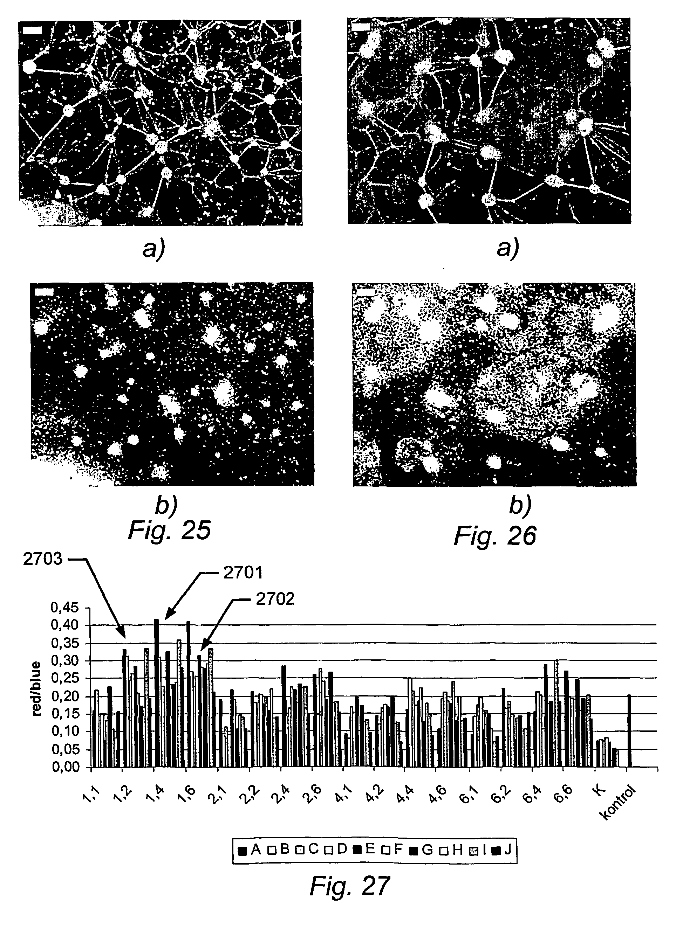

Fig. 22 shows cells on a control surface (no structure). Fig. 23 shows cells on structure A1.4 (as described in connection with fig. 12a). Fig. 24 shows cells on structure A2.1 (as described in connection with fig. 12a). Fig. 25 shows cells on structure B1.4 (as described in connection with fig. 12b). Fig. 26 shows cells on structure B2.1 (as described in connection with fig. 12b).

A major part of the cells are positive for β-tubulin isoform III, indicating that they are neurons (see e.g. Fig. 22). On most structures as well as on the control there are areas with high density of cells that are not stained with anti-β-tubulin III or that only stain very weakly, indicating that they are not neurons (Fig. 22, Fig. 24, and Fig. 26). In particular, Fig. 22 shows that on the unstructured surface, the cell density is high but there are "plaques" of cells that are not stained (indicated by black arrows in Fig. 22b) or stained very weakly (indicated by black arrowheads in Fig. 22b) with anti-β-tubulin III.

In contrast, on some of the structures, (e.g. A1.4 and B1.4, see Fig. 23 and Fig. 25, respectively), no such plaques of non-neuronal cells are seen, and a higher proportion of the cells have become neurons.

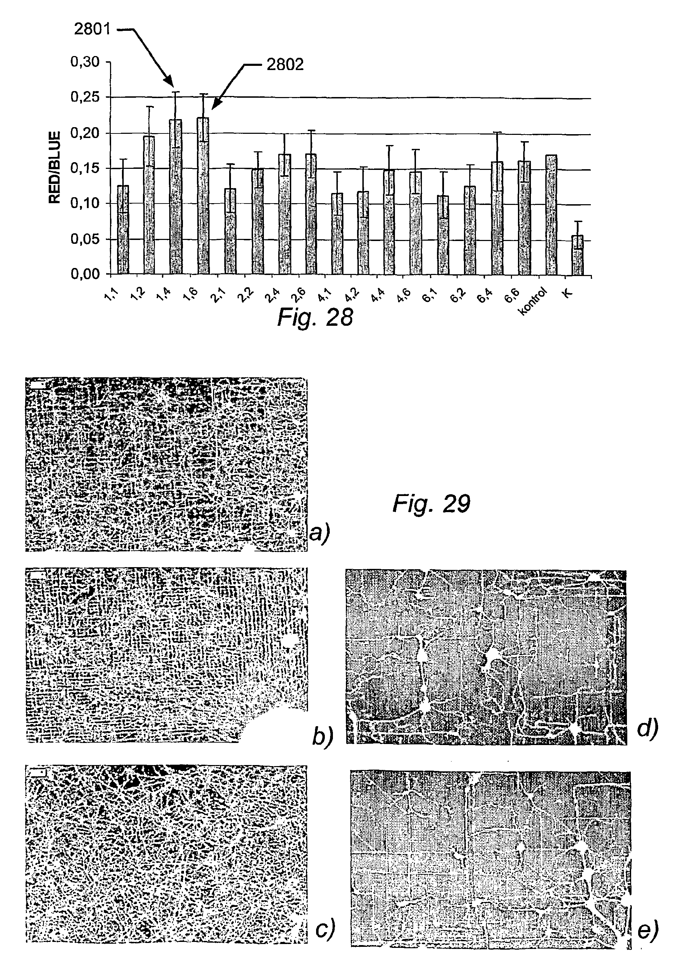

To quantify neuronal differentiation ratio, the area of red and blue staining was determined and the area of red staining was divided by the area of blue staining so as to obtain a measure of neuronal differentiation. The results of these measurements are shown in Fig. 27 for different types of structures and different structure sizes. For all the structures of series A to J (as described in connection with figs. 12a-j), a higher neuronal differentiation proportion is seen on the structures with the sizes 1,4 (reference sign 2701) and 1,6 (reference sign 2702) and to some extent also 1,2 (reference sign 2703), indicating that both the size and spacing of the features/pillars are important. Among the structures tested a cross-sectional feature size of about one micrometer and a minimum inter-feature spacing between adjacent/nearest-neighbor features of between 4 to 6 micrometer has been found to be particularly advantageous. When the results are pooled according to the structure sizes, as shown in Fig. 28, it can be seen that neuronal differentiation is significantly stimulated by the structures with sizes 1,4 (reference sign 2801) and 1,6 (reference sign 2802). In contrast, the K structures (sharkskin) have a significant lower proportion of neuronal cells (beta-tubulin III-postive cells) than all other structures including the control surface (all K-structures were pooled).

### Example 9

### Directed growth of neurites

Primary neuronal culture from cortex from 18 days old rat embryos were established on a BSSA wafer and after 7 days in vitro, the cells are fixed and stained with antibody against neuronal specific β-tubulin III.

Fig. 29 shows pictures of primary neuronal culture stained with anti-β-tubulin III antibody on different structures as described in connection with fig. 12. In particular, Fig. 29a shows structure C1.2, Fig. 29b shows structure C6.4, Fig. 29c shows a control surface (no structure), Fig. 29d shows structure C4.2, and Fig. 29e shows structure F1.2.

As can be seen from Fig. 29, some of the structures clearly guide the neurite outgrowth in defined directions. Again it was found that the sizes of the structures tend to be more important than the actual structure pattern. In general the degree in which the structures guide neurites can be represented as follows: 1,1 < 1,2 > 1,4; 2,1 < 2,2 - 2,4; 4,2 - 4,4; 6,2 < 6,4 > 6,6. In contrast, structures with large pillars and small spaces or small pillars and large spaces do not function to guide outgrowth.

### Example 10

### Mineralisation of human mesenchymal stem cells on BSSA wafers FH001

Bone marrow samples were aspirated from the posterior superior iliac spine. The low-density mononuclear cells were isolated by gradient centrifugation with Lymphoprep®. The purified cells from 3 donors were mixed and grown in culture flasks for 2 passages before the 3rd passage was seeded at a concentration of 18,000 cells/cm² onto a BSSA wafer. Fresh medium (MEM [Earles] without phenol red containing 10% fetal calf serum [FCS], 100U/ml penicillin, and 100 microgram/ml streptomycin was added the next day. After a week, medium with 284 µM ascorbic acid, 10 mM β-glycerophosphate, and 10 nM dexamethasone was given for stimulation of the-cells. The medium was changed once every week. After 4½ weeks, the wafer was stained with Alizarin Red.

The following structures showed increased mineralisation (classified as ranged from 1 to 4 with decreasing intensity):
Range 1: F4.2; E6.2;
Range 2: H1.1; D1.1; B2.1.
Range 3: I1.1; G1.1; K1; K2; K4; K5; K6.
Range 4: K3; K7; K8; J2.1.

Hence, generally structures with features of different cross sectional geometries (such as E*X*.*Y*; F*X*.*Y*; H*X*.*Y*; I*X*.*Y*; J*X*.*Y*) and/or structures where not all grid points are filled with protrusions forming hexagonal areas surrounded by protrusions (such as B*X*.*Y*; D*X*.*Y*; GX.*Y*; J*X*.*Y)* and/or the "sharkskin" structures (K structures) and/or structures with cross-sectional feature dimension of around 1 µm (*X*=1) and centre-to-centre distances between adjacent features of about 2-3µm (X+Y between 2 and 3).

## Claims

1. A method of promoting growth of undifferentiated embryonic stem cells, the method comprising bringing the cells into contact with a biocompatible material, wherein at least a part of a surface of the biocompatible material is **characterized by** a nano- or micrometer scale topographical structure comprising a plurality of features arranged in a regular pattern where the structure is selected to promote growth of undifferentiated embryonic stem cells, wherein each of the features has at least one lateral dimension between about 0.1-20µm.

2. A method of promoting neuronal differentiation of embryonic stem cells, the method comprising bringing the cells into contact with a biocompatible material, wherein at least a part of a surface of the biocompatible material is **characterized by** a nano- or micrometer scale topographical structure comprising a plurality of features arranged in a regular pattern where the structure is selected to promote neuronal differentiation of embryonic stem cells, wherein each of the features has at least one lateral dimension between about 0.1-20 µm.

3. A method according to claim 1 or 2, wherein at least one lateral dimension of any one of said features is between about 0.5 µm and about 2 µm, preferably between about 0.8 µm and about 1.2 µm, more preferably between about 0.9 µm and about 1.1 µm, e.g. about 1 µm.

4. A method according to any one of claims 1 - 3, wherein said features are arranged in a regular pattern having a minimum gap size between adjacent features of between about 1 µm and about 7µm, preferably between about 2µm and about 6µm.

5. A method according to any one of claims 2-4, wherein the features include protrusions regularly arranged so as to generate a pattern where respective pluralities of protrusions are arranged so as to surround a corresponding area without protrusions, the area without protrusions having a linear dimension larger than the minimum inter feature gap size, preferably larger than twice the minimum inter-feature gap size.

6. A method of promoting differentiation of embryonic stem cells, the method comprising bringing the cells into contact with a biocompatible material, wherein at least a part of a surface of the biocompatible material is **characterized by** a nano- or micrometer scale topographical structure comprising a plurality of features arranged in a regular pattern where the structure is selected to promote differentiation of embryonic stem cells, wherein each of the features has at least one lateral dimension between about 0.1-20µm.

7. A method according to claim 6, wherein the structure includes a plurality of elongated ridges arranged in a regular pattern.

8. A method according to claim 7, wherein the elongated ridges have different lengths and are arranged in a regular pattern.

9. A method of promoting outgrowth of neurites from primary neuronal cells in defined directions, the method comprising bringing the cells into contact with a biocompatible material, wherein at least a part of a surface of the biocompatible material is **characterized by** a nano- or micrometer scale topographical structure comprising a plurality of features arranged in a regular pattern where the structure is selected to promote outgrowth of neurites from primary neuronal cells in defined directions, wherein each of the features has at least one lateral dimension between about 0.1-20µm.

10. A method according to claim 9, wherein the features are arranged in a regular pattern having minimum gap size between adjacent features of between about 1 µm and about 5 µm, preferably between about 2 µm and about 4 µm.

11. A method according to claim 10, wherein at least one lateral dimension of any one of said features is between about 0.5 µm and about 1.5 µm, and the minimum gap size between adjacent features is between about 1 µm and about 6 µm.

12. A method according to claim 11, wherein at least one lateral dimension of any one of said features is between about 1.5 µm and about 2.5 µm, and the minimum gap size between adjacent features is between about 1 µm and about 4 µm.

13. A method according to claim 11, wherein at least one lateral dimension of any one of said features is between about 3.5 µm and about 4.5 µm, and the minimum gap size between adjacent features is between about 1.5 µm and about 4.5 µm.

14. A method according to claim 11, wherein at least one lateral dimension of any one of said features is between about 5.5 µm and about 6.5 µm, and the minimum gap size between adjacent features is between about 2 µm and about 6 µm.

15. A method according to any one of claims 1 through 11, where each of the features has at least one lateral dimension (X) in at least one of the intervals between about 1-10µm and between about 10-20µm.

16. A method according to claim 15, wherein the lateral dimension (X) is selected from one of the intervals: between about 1 µm - 2 µm, between about 2µm - 4 µm; between about 4µm - 6 µm; between about 6µm - 8 µm; between about 8µm - 10 µm; between about 10-12µm; between about 12-14µm; between about 14-16µm; between about 16-18µm; between about 18-20µm.

17. A method according to any one of claims 1 through 16, wherein each feature has a cross-sectional area such that the shortest distance from any point within said cross-sectional area to an edge of the cross-sectional area is no more than 10µm.

18. A method according to any one of claims 1 through 17, wherein the biocompatible material further comprises an adsorbed compound selected from the group consisting of: polypeptide, carbohydrate, lipid, growth hormone, antibody, antigen, glycoprotein, lipoprotein, DNA, RNA, polysaccharide, lipid, organic compound, and inorganic compound.

19. A method according to claim 18, wherein said growth hormone is selected from the group consisting of BMP, EGF-like, TGF-beta.

20. A method according to any one of claims 1 through 19, wherein the protrusions have a cross section with a minimum cross-sectional diameter no larger than 2 µm, preferably no larger than 1.5 µm and wherein the cross-sectional diameter is larger than 10nm, such as larger than 50nm, such as larger than 100nm, such as between 0.1 µm and 2 µm, such as between 0.5 µm and 2µm, such as between 0.1 µm and 1.5µm, such as between 0.5 µm and 1.5µm.

21. A method according to any one of claims 1 through 20, wherein a maximum cross-sectional diameter of the cross section is no larger than 2 µm, preferably between 0.01 µm and 2 µm, preferably between 0.1 µm and 2 µm, preferably between 0.5 µm and 2µm, such as between 0.1 µm and 1.5µm, such as between 0.5 µm and 1.5µm.

22. A method according to any one of claims 1 through 21, wherein the distance between adjacent grid points along at least one dimension is no larger than 7µm.

23. A method according to claim 22, wherein the distance between adjacent grid points along at least one dimension is smaller than 4µm, such as between 0.01 µm and 4 µm, preferably 0.1 µm and 4 µm, more preferably between 0.5 µm and 3.5µm, e.g. between 1 µm and 3µm.

24. A method according to claim 22 or 23, wherein the distance between adjacent grid points along the two dimensions is no larger than 4µm, preferably between 0.01 µm and 4 µm, preferably between 0.1 µm and 4 µm more preferably between 0.5 µm and 3.5µm, such as between 1µm and 3µm.

25. A method according to any one of claims 1 through 24, wherein the structure includes protrusions of at least two different cross-sectional geometrical shapes.

26. A method according to claim 25, wherein the protrusions of different cross sectional geometry are arranged on the regular two-dimensional grid in an alternating pattern.

27. A method according to any one of claims 1 through 26, wherein the structure includes protrusions of different cross-sectional area.

28. A method according to claim 27, wherein the protrusions are elongated ridges having different lengths.

29. A method according to claim 28, wherein the elongated ridges each have a width of between 0.1 µm and 2 µm, preferably between 0.5 µm and 1.5 µm.

30. A method according to claim 28 or 29, wherein the distance between adjacent elongated ridges is smaller than 2 µm, preferably between 0.1 µm and 2 µm, preferably between 0.5 µm and 1.5 µm.

31. A method according to any one of claims 28 through 30, wherein the respective lengths of the elongated ridges is smaller than 20µm, preferably smaller than 10µm, e.g. between 0.5µm and 10µm.

32. A method according to any one of claims 1 through 31, wherein the protrusions are positioned on grid points of the two-dimensional regular grid such that only a subset of grid points are covered by protrusions.

33. A method according to any one of claims 1 through 32, wherein the protrusions are arranged in parallel rows where the centre-to-centre distance between adjacent protrusions is different in adjacent rows.

34. A method according to any one of claims 1 through 33, wherein the lateral cross-section of one or more feature has a shape defined by circumference and/or geometry selected from one the shapes: circular, round, star, square, rectangular, hexagonal and polygonal or a combination thereof.

35. A method according to any one of claims 1 through 34, wherein one or more feature has a generally square cross-section.

36. A method according to claim 35, wherein one or more feature has a generally circular cross-section and one or more feature has a generally square cross-section.

37. A method according to any one of claims 1 through 36, wherein the lateral dimension of the maximum gap between any feature and its nearest neighbor (d;Y) is within at least one of the intervals: between about 0.5 µm - 1.0 µm, between about 1 µm - 2 µm, between about 2µm - 4 µm, between about 4µm - 6 µm, between about 8 µm -10 µm, between about 10 µm - 12 µm, between about 12 µm -14µm, between about 14 µm - 16 µm.

38. A method according to any one of claims 1 through 37, wherein the surface of the material is **characterized by** a periodic micrometer scale topographical structure whose lateral pitch dimension in any lateral dimension is selected from at least one of the intervals: between about 1 µm - 2 µm; between about 2µm - 4 µm, between about 4µm - 6 µm between about 6µm - 10 µm, between about 10µm - 16 µm, between about 16 µm -20 µm, between about 20 µm -24 µm.

39. A method according to any one of claims 1 through 38, wherein each of the features of said topographical structure has a vertical height/depth dimension selected from at least one of the intervals: of between about 1 nm - 0.1 µm, of between about 0.1 µm - 0.5 µm, of between about 0.07 µm - 1.6 µm, of between about 1.6 µm - 3.0 µm, between about 3µm - 10 µm.

40. A method according to any one of claims 1 through 39, wherein the center of the features of said periodic topographical structure are placed on grid points of a 2-dimensional rectangular grid with grid constants a and b, and wherein:
a. the grid is a square grid wherein the grid constant in each direction (a=b) is in an interval between 2 -12µm, or
b. the grid is rectangular with a grid constant (a) in a first direction in an interval between 2 - 12 µm and with a grid constant (b) in a second direction in an interval between 1- 6 µm, between about 6 µm - 10 µm, between about 10 µm - 16 µm, between about 16 µm -20 µm, between about 20 µm -24 µm.

41. A method according to any one of claims 1 through 40, wherein at least a part of said surface is tantalum-coated and/or titanium-coated.

42. A method according to any one of claims 1 through 41, wherein at least some of the features have a top surface having a topographical structure on a nano scale.

## Patentansprüche

1. Verfahren zur Förderung von Wachstum undifferenzierter embryonaler Stammzellen, welches Verfahren das Inkontaktbringen der Zellen mit einem biokompatiblen Material umfasst, wobei mindestens ein Teil einer Fläche des biokompatiblen Materials durch eine topographische Struktur auf einer Nano- oder Mikrometerskala **gekennzeichnet** ist, umfassend eine Mehrzahl von in einem regulären Muster angebrachten Bestandteilen, wobei die Struktur zur Förderung von Wachstum undifferenzierter embryonaler Stammzellen ausgewählt wird, wobei jeder der Bestandteile mindestens eine laterale Dimension zwischen etwa 0,1-20 µm aufweist.

2. Verfahren zur Förderung von neuronaler Differenzierung embryonaler Stammzellen, welches Verfahren das Inkontaktbringen der Zellen mit einem biokompatiblen Material umfasst, wobei mindestens ein Teil einer Fläche des biokompatiblen Materials durch eine topographische Struktur auf einer Nano- oder Mikrometerskala **gekennzeichnet** ist, umfassend eine Mehrzahl von in einem regulären Muster angebrachten Bestandteilen, wobei die Struktur zur Förderung von neuronaler Differenzierung embryonaler Stammzellen ausgewählt wird, wobei jeder der Bestandteile mindestens eine laterale Dimension zwischen etwa 0,1-20 µm aufweist.

3. Verfahren nach Anspruch 1 oder 2, wobei mindestens eine laterale Dimension nach irgendeinem der Bestandteile zwischen etwa 0,5 µm und etwa 2 µm, bevorzugt zwischen etwa 0,8 µm und etwa 1,2 µm, mehr bevorzugt zwischen etwa 0,9 µm und etwa 1,1 µm, z.B. etwa 1 µm, liegt.

4. Verfahren nach irgendeinem der Ansprüche 1-3, wobei die Bestandteile in einem regulären Muster angebracht werden, das eine Mindestspaltgröße zwischen nebeneinanderliegenden Bestandteilen von zwischen etwa 1 µm und etwa 7 µm, bevorzugt zwischen etwa 2 µm und etwa 6 µm aufweist.

5. Verfahren nach irgendeinem der Ansprüche 2-4, wobei die Bestandteile Vorsprünge enthalten, die regulär angebracht werden, um ein Muster zu erzeugen, wobei jeweilige mehrere Vorsprünge zur Umschließung eines entsprechenden Bereichs ohne Vorsprünge vorgesehen werden, welcher Bereich ohne Vorsprünge eine lineare Dimension aufweist, die größer als die Mindest-Zwischenbestandteil-Spaltgröße, bevorzugt größer als das Doppelte der Mindest-Zwischenbestandteil-Spaltgröße, ist.

6. Verfahren zur Förderung von Differenzierung embryonaler Stammzellen, welches Verfahren das Inkontaktbringen der Zellen mit einem biokompatiblen Material umfasst, wobei mindestens ein Teil einer Fläche des biokompatiblen Materials durch eine topographische Struktur auf einer Nano- oder Mikrometerskala **gekennzeichnet** ist, umfassend eine Mehrzahl von in einem regulären Muster angebrachten Bestandteilen, wobei die Struktur zur Förderung von Differenzierung embryonaler Stammzellen ausgewählt wird, wobei jeder der Bestandteile mindestens eine laterale Dimension zwischen etwa 0,1-20 µm aufweist.

7. Verfahren nach Anspruch 6, wobei die Struktur eine Mehrzahl von länglichen in einem regulären Muster angebrachten Kämmen enthält.

8. Verfahren nach Anspruch 7, wobei die länglichen Kämme verschiedene Längen aufweisen und in einem regulären Muster angebracht werden.

9. Verfahren zur Förderung von Auswuchs von Neuriten aus primären neuronalen Zellen in definierten Richtungen, welches Verfahren das Inkontaktbringen der Zellen mit einem biokompatiblen Material umfasst, wobei mindestens ein Teil einer Fläche des biokompatiblen Materials durch eine topographische Struktur auf einer Nano- oder Mikrometerskala **gekennzeichnet** ist, umfassend eine Mehrzahl von in einem regulären Muster angebrachten Bestandteilen, wobei die Struktur zur Förderung von Auswuchs von Neuriten aus primären neuronalen Zellen in definierten Richtungen ausgewählt wird, wobei jeder der Bestandteile mindestens eine laterale Dimension zwischen etwa 0,1-20 µm aufweist.

10. Verfahren nach Anspruch 9, wobei die Bestandteile in einem regulären Muster angebracht werden, das eine Mindestspaltgröße zwischen nebeneinanderliegenden Bestandteilen von zwischen etwa 1 µm und etwa 5 µm, bevorzugt zwischen etwa 2 µm und etwa 4 µm aufweist.

11. Verfahren nach Anspruch 10, wobei mindestens eine laterale Dimension nach irgendeinem der Bestandteile zwischen etwa 0,5 µm und etwa 1,5 µm liegt, und die Mindestspaltgröße zwischen nebeneinanderliegenden Bestandteilen zwischen etwa 1 µm und etwa 6 µm liegt.

12. Verfahren nach Anspruch 11, wobei mindestens eine laterale Dimension nach irgendeinem der Bestandteile zwischen etwa 1,5 µm und etwa 2,5 µm liegt, und die Mindestspaltgröße zwischen nebeneinanderliegenden Bestandteilen zwischen etwa 1 µm und etwa 4 µm liegt.

13. Verfahren nach Anspruch 11, wobei mindestens eine laterale Dimension nach irgendeinem der Bestandteile zwischen etwa 3,5 µm und etwa 4,5 µm liegt, und die Mindestspaltgröße zwischen nebeneinanderliegenden Bestandteilen zwischen etwa 1,5 µm und etwa 4,5 µm liegt.

14. Verfahren nach Anspruch 11, wobei mindestens eine laterale Dimension nach irgendeinem der Bestandteile zwischen etwa 5,5 µm und etwa 6,5 µm liegt, und die Mindestspaltgröße zwischen nebeneinanderliegenden Bestandteilen zwischen etwa 2 µm und etwa 6 µm liegt.

15. Verfahren nach irgendeinem der Ansprüche 1 bis 11, wobei jeder der Bestandteile mindestens eine laterale Dimension (X) in mindestens einem der Intervalle zwischen etwa 1-10 µm und zwischen etwa 10-20 µm aufweist.

16. Verfahren nach Anspruch 15, wobei die laterale Dimension (X) aus einem der Intervalle: zwischen etwa 1 µm-2 µm, zwischen etwa 2 µm-4 µm; zwischen etwa 4 µm-6 µm; zwischen etwa 6 µm-8 µm; zwischen etwa 8 µm-10 µm; zwischen etwa 10-12 µm; zwischen etwa 12-14 µm; zwischen etwa 14-16 µm; zwischen etwa 16-18 µm; zwischen etwa 18-20 µm ausgewählt wird.

17. Verfahren nach irgendeinem der Ansprüche 1 bis 16, wobei jeder Bestandteil einen Querschnittsbereich aufweist, so dass der kürzeste Abstand von irgendeinem Punkt innerhalb des Querschnittsbereichs zu einem Rand des Querschnittsbereichs nicht mehr als 10 µm beträgt.

18. Verfahren nach irgendeinem der Ansprüche 1 bis 17, wobei das biokompatible Material außerdem eine adsorbierte Verbindung umfasst, die aus der Gruppe ausgewählt wird, die aus: Polypeptid, Carbohydrat, Lipid, Wachstumshormon, Antikörper, Antigen, Glycoprotein, Lipoprotein, DNA, RNA, Polysaccharid, Lipid, organischer Verbindung und anorganischer Verbindung besteht.

19. Verfahren nach Anspruch 18, wobei das Wachstumshormon aus der aus BMP, EGF-Ähnlichem, TGF-Beta bestehenden Gruppe ausgewählt wird.

20. Verfahren, nach irgendeinem der Ansprüche 1 bis 19, wobei die Vorsprünge einen Querschnitt mit einem Mindestquerschnittsdurchmesser nicht größer als 2 µm, bevorzugt nicht größer als 1,5 µm, aufweisen, und wobei der Querschnittsdurchmesser größer als 10 nm ist; wie etwa größer als 50 nm, wie etwa größer als 100 nm, wie etwa zwischen 0,1 µm und 2 µm, wie etwa zwischen 0,5 µm und 2 µm, wie etwa zwischen 0,1 µm und 1,5 µm, wie etwa zwischen 0,5 µm und 1,5 µm.

21. Verfahren nach irgendeinem der Ansprüche 1 bis 20, wobei ein maximaler Querschnittsdurchmesser des Querschnitts nicht größer als 2 µm, bevorzugt zwischen 0,01 µm und 2 µm, bevorzugt zwischen 0,1 µm und 2 µm, bevorzugt zwischen 0,5 µm und 2 µm, wie etwa zwischen 0,1 µm und 1,5 µm, wie etwa zwischen 0,5 µm und 1,5 µm, ist.

22. Verfahren nach irgendeinem der Ansprüche 1 bis 21, wobei der Abstand zwischen nebeneinanderliegenden Gitterpunkten entlang mindestens einer Dimension nicht größer als 7 µm ist.

23. Verfahren nach Anspruch 22, wobei der Abstand zwischen nebeneinanderliegenden Gitterpunkten entlang mindestens einer Dimension kleiner als 4 µm, wie etwa zwischen 0,01 µm und 4 µm, bevorzugt 0,1 µm und 4 µm, mehr bevorzugt zwischen 0,5 µm und 3,5 µm, z.B. zwischen 1 µm und 3 µm, ist.

24. Verfahren nach Anspruch 22 oder 23, wobei der Abstand zwischen nebeneinanderliegenden Gitterpunkten entlang den beiden Dimensionen nicht größer als 4 µm, bevorzugt zwischen 0,01 µm und 4 µm, bevorzugt zwischen 0,1 µm und 4 µm, mehr bevorzugt zwischen 0,5 µm und 3,5 µm, wie etwa zwischen 1 µm und 3 µm, ist.

25. Verfahren nach irgendeinem der Ansprüche 1 bis 24, wobei die Struktur Vorsprünge von mindestens zwei verschiedenen querschnittlichen geometrischen Formen enthält.

26. Verfahren nach Anspruch 25, wobei die Vorsprünge einer verschiedenen Querschnittsgeometrie auf dem regulären zweidimensionalen Gitter in einem wechselnden Muster angebracht werden.

27. Verfahren nach irgendeinem der Ansprüche 1 bis 26, wobei die Struktur Vorsprünge eines verschiedenen Querschnittsbereichs enthält.

28. Verfahren nach Anspruch 27, wobei die Vorsprünge verschiedene Längen aufweisende längliche Kämme sind.

29. Verfahren nach Anspruch 28, wobei die länglichen Kämme jeweils eine Breite von etwa 0,1 µm und 2 µm, bevorzugt zwischen 0,5 µm und 1,5 µm, aufweisen.

30. Verfahren nach Anspruch 28 oder 29, wobei der Abstand zwischen nebeneinanderliegenden länglichen Kämmen kleiner als 2 µm, bevorzugt zwischen 0,1 µm und 2 µm, bevorzugt zwischen 0,5 µm und 1,5 µm, ist.

31. Verfahren nach irgendeinem der Ansprüche 28 bis 30, wobei die jeweiligen Längen der länglichen Kämme kleiner als 20 µm, bevorzugt kleiner als 10 µm, z.B. zwischen 0,5 µm und 10 µm, sind.

32. Verfahren nach irgendeinem der Ansprüche 1 bis 31, wobei die Vorsprünge in Gitterpositionen des zweidimensionalen regulären Gitters so positioniert werden, dass nur eine Teilmenge von Gitterpunkten durch Vorsprünge abgedeckt wird.

33. Verfahren nach irgendeinem der Ansprüche 1 bis 32, wobei die Vorsprünge in parallelen Reihen angeordnet werden, in denen der Mittenabstand zwischen nebeneinanderliegenden Vorsprüngen in nebeneinanderliegenden Reihen verschieden ist.

34. Verfahren nach irgendeinem der Ansprüche 1 bis 33, wobei der laterale Querschnitt eines oder mehrerer Bestandteile eine durch einen Umfang und/oder eine Geometrie definierte Form aufweist, ausgewählt aus einer der Formen: kreisförmig, rund, sternförmig, quadratisch, rechteckig, hexagonal und polygonal oder eine Kombination davon.

35. Verfahren nach irgendeinem der Ansprüche 1 bis 34, wobei ein oder mehrere Bestandteile einen im Allgemeinen quadratischen Querschnitt aufweist/aufweisen.

36. Verfahren nach Anspruch 35, wobei ein oder mehrere Bestandteile einen im Allgemeinen kreisförmigen Querschnitt aufweisen, und ein oder mehrere Bestandteile einen im Allgemeinen quadratischen Querschnitt aufweist/aufweisen.

37. Verfahren nach irgendeinem der Ansprüche 1 bis 36, wobei sich die laterale Dimension des maximalen Spalts zwischen irgendeinem Bestandteil und dessen nächsten Nachbarn (d;Y) innerhalb mindestens einem der Intervalle: zwischen etwa 0,5 µm-1,0 µm, zwischen etwa 1 µm-2 µm, zwischen etwa 2 µm-4 µm, zwischen etwa 4 µm-6 µm, zwischen etwa 8 µm-10 µm, zwischen etwa 10 µm-12 µm, zwischen etwa 12 µm-14 µm, zwischen etwa 14 µm-16 µm befindet.

38. Verfahren nach irgendeinem der Ansprüche 1 bis 37, wobei die Fläche des Materials durch eine topographische Struktur auf einer periodischen Mikrometerskala **gekennzeichnet** ist, deren laterale Pitch-Dimension in irgendeiner lateralen Dimension aus mindestens einem der Intervalle: zwischen etwa 1 µm-2 µm; zwischen etwa 2 µm-4 µm, zwischen etwa 4 µm - 6 µm, zwischen etwa 6 µm-10 µm, zwischen etwa 10 µm-16 µm, zwischen etwa 16 µm-20 µm, zwischen etwa 20 µm-24 µm ausgewählt wird.

39. Verfahren nach irgendeinem der Ansprüche 1 bis 38, wobei jeder der Bestandteile der topographischen Struktur eine vertikale Höhen-/Tiefendimension aufweist, ausgewählt aus mindestens einem der Intervalle: von zwischen etwa 1 nm-0,1 µm, von zwischen etwa 0,1 µm-0,5 µm, von zwischen etwa 0,07 µm-1,6 µm, von zwischen etwa 1,6 µm-3,0 µm, zwischen etwa 3 µm-10 µm.

40. Verfahren nach irgendeinem der Ansprüche 1 bis 39, wobei das Zentrum der Bestandteile der periodischen topographischen Struktur auf Gitterpunkten eines zweidimensionalen rechteckigen Gitters mit Gitterkonstanten a und b angebracht wird, und wobei:
a. das Gitter ein quadratisches Gitter ist, wobei sich die Gitterkonstante in jeder Richtung (a=b) in einem Intervall zwischen 2-12 µm befindet, oder
b. das Gitter mit einer Gittekonstante (a) in einer ersten Richtung in einem Intervall zwischen 2-12 µm und mit einer Gitterkonstante (b) in einer zweiten Richtung in einem Intervall zwischen 1-6 µm, zwischen etwa 6 µm-10 µm, zwischen etwa 10 µm-16 µm, zwischen etwa 16 µm-20 µm, zwischen etwa 20 µm-24 µm rechteckig ist.

41. Verfahren nach irgendeinem der Ansprüche 1 bis 40, wobei zumindest ein Teil der Fläche tantalbeschichtet und/oder titaniumbeschichtet wird.

42. Verfahren nach irgendeinem der Ansprüche 1 bis 41, wobei zumindest einige der Bestandteile eine eine topographische Struktur auf einer Nanoskala aufweisende Oberfläche aufweisen.

## Revendications

1. Procédé pour la promotion de la croissance de cellules souches embryonnaires indifférenciées, procédé qui comprend la mise en contact des cellules avec un matériau biocompatible, dans lequel au moins une partie d'une surface du matériau biocompatible est **caractérisée par** une structure topographique à l'échelle nano- ou micrométrique comprenant une pluralité de caractéristiques arrangées dans un motif régulier, la structure étant sélectionnée pour la promotion de la croissance de cellules souches embryonnaires indifférenciées, chacune des caractéristiques présentant au moins une dimension latérale comprise entre environ 0,1 et 20 µm.

2. Procédé pour la promotion de la différenciation neuronale de cellules souches embryonnaires, procédé qui comprend la mise en contact des cellules avec un matériau biocompatible, dans lequel au moins une partie d'une surface du matériau biocompatible est **caractérisée par** une structure topographique à l'échelle nano- ou micrométrique comprenant une pluralité de caractéristiques arrangées dans un motif régulier, la structure étant sélectionnée pour la promotion de la différenciation neuronale de cellules souches embryonnaires, chacune des caractéristiques présentant au moins une dimension latérale comprise entre environ 0,1 et 20 µm.

3. Procédé selon la revendication 1 ou 2, dans lequel au moins une dimension latérale de chacune desdites caractéristiques est comprise entre environ 0,5 µm et environ 2 µm, préférablement entre environ 0,8 µm et environ 1,2 µm, plus préférablement entre environ 0,9 µm et environ 1,1 µm, p. ex. environ 1 µm.

4. Procédé selon l'une quelconque des revendications 1 à 3, dans lequel lesdites caractéristiques sont arrangées dans un motif régulier présentant des dimensions minimales d'écartement entre des caractéristiques adjacentes comprises entre environ 1 µm et environ 7 µm, préférablement entre environ 2 µm et environ 6 µm.

5. Procédé selon l'une quelconques des revendications 2 à 4, dans lequel les caractéristiques comprennent des saillies régulièrement arrangées pour générer un motif dans lequel des pluralités respectives de saillies sont disposées de manière à entourer une superficie correspondante sans saillies, la superficie sans saillies présentant une dimension linéaire et plus large que les dimensions minimales d'écartement entre les caractéristiques, préférablement plus large que deux fois les dimensions minimales d'écartement entre les caractéristiques.

6. Procédé pour la promotion de la différenciation de cellules souches embryonnaires, procédé qui comprend la mise en contact des cellules avec un matériau biocompatible, dans lequel au moins une partie d'une surface du matériau biocompatible est **caractérisée par** une structure topographique à l'échelle nano- ou micrométrique comprenant une pluralité de caractéristiques arrangées dans un motif régulier, la structure étant sélectionnée pour la promotion de la différenciation de cellules souches embryonnaires, chacune des caractéristiques présentant au moins une dimension latérale comprise entre environ 0,1 et 20 µm.

7. Procédé selon la revendication 6, dans lequel la structure comprend une pluralité de crêtes allongées et arrangées dans un motif régulier.

8. Procédé selon la revendication 7, dans lequel les crêtes allongées présentent des longueurs différentes et sont arrangées dans un motif régulier.

9. Procédé pour la promotion de l'excroissance de neurites de cellules neuronales primaires dans des directions définies, procédé qui comprend la mise en contact des cellules avec un matériau biocompatible, dans lequel au moins une partie d'une surface du matériau biocompatible est **caractérisée par** une structure topographique à l'échelle nano- ou micrométrique comprenant une pluralité de caractéristiques arrangées dans un motif régulier, la structure étant sélectionnée pour la promotion de l'excroissance de neurites de cellules neuronales primaires dans des directions définies, chacune des caractéristiques présentant au moins une dimension latérale comprise entre environ 0,1 et 20 µm.

10. Procédé selon la revendication 9, dans lequel les caractéristiques sont arrangées dans un motif régulier présentant des dimensions minimales d'écartement entre des caractéristiques adjacentes comprises entre environ 1 µm et environ 5 µm, préférablement entre environ 2 µm et environ 4 µm.

11. Procédé selon la revendication 10, dans lequel au moins une dimension latérale de chacune desdites caractéristiques est comprise entre environ 0,5 µm et environ 1,5 µm, et les dimensions minimales d'écartement entre des caractéristiques adjacentes sont comprises entre environ 1 µm et environ 6 µm.

12. Procédé selon la revendication 11, dans lequel au moins une dimension latérale de chacune desdites caractéristiques est comprise entre environ 1,5 µm et environ 2,5 µm, et les dimensions minimales d'écartement entre des caractéristiques adjacentes sont comprises entre environ 1 µm et environ 4 µm.

13. Procédé selon la revendication 11, dans lequel au moins une dimension latérale de chacune desdites caractéristiques est comprise entre environ 3,5 µm et environ 4,5 µm, et les dimensions minimales d'écartement entre des caractéristiques adjacentes sont comprises entre environ 1,5 µm et environ 4,5 µm.

14. Procédé selon la revendication 11, dans lequel au moins une dimension latérale de chacune desdites caractéristiques est comprise entre environ 5,5 µm et environ 6,5 µm, et les dimensions minimales d'écartement entre des caractéristiques adjacentes sont comprises entre environ 2 µm et environ 6 µm.

15. Procédé selon l'une quelconque des revendications 1 à 11, dans lequel chacune des caractéristiques présente au moins une dimension latérale (X) dans au moins l'un des intervalles compris entre environ 1 et 10 µm et entre environ 10 et 20 µm.

16. Procédé selon la revendication 15, dans lequel la dimension latérale (X) est sélectionnée parmi l'un des intervalles compris: entre environ 1 µm et 2 µm, entre environ 2 µm et 4 µm; entre environ 4 µm et 6 µm; entre environ 6 µm et 8 µm; entre environ 8 µm et 10 µm; entre environ 10 µm et 12 µm; entre environ 12 µm et 14 µm; entre environ 14 µm et 16 µm; entre environ 16 µm et 18 µm ; entre environ 18 µm et 20 µm.

17. Procédé selon l'une quelconque des revendications 1 à 16, dans lequel chaque caractéristique présente une superficie de section transversale si bien que la distance la plus courte à partir de tout point dans la superficie de section transversale à un bord de la superficie de section transversale n'excède pas 10 µm.

18. Procédé selon l'une quelconque des revendications 1 à 17, dans lequel le matériau biocompatible en outre comprend un composé adsorbé sélectionné parmi le groupe comprenant: polypeptide, hydrate de carbone, lipide, hormone de croissance, anticorps, antigène, glycoprotéine, lipoprotéine, ADN, ARN, polysaccharide, lipide, composé organique et composé inorganique.

19. Procédé selon la revendication 18, dans lequel ladite hormone de croissance est sélectionnée parmi le groupe comprenant PMO (Protéine de la morphogenèse osseuse) (BMP), semblant à l'EGF (au facteur de croissance épidermique), TGF-bêta.

20. Procédé selon l'une quelconque des revendications 1 à 19, dans lequel les saillies présentent une section transversale avec un diamètre de section transversale n'excédant pas 2 µm, préférablement n'excédant pas 1,5 µm, et dans lequel le diamètre de section transversale est supérieur à 10 nm, tel que supérieur à 50 nm, tel que supérieur à 100 nm, tel qu'entre 0,1 µm et 2 µm, tel qu'entre 0,5 µm et 2 µm, tel qu'entre 0,1 µm et 1,5 µm, tel qu'entre 0,5 µm et 1,5 µm.

21. Procédé selon l'une quelconque des revendications 1 à 20, dans lequel un diamètre maximal de section transversale de la coupe transversale n'excède pas 2 µm, préférablement entre 0,01 µm et 2 µm, préférablement entre 0,1 µm et 2 µm, préférablement entre 0,5 µm et 2 µm, tel qu'entre 0,1 µm et 1,5 µm, tel qu'entre 0,5 µm et 1,5 µm.

22. Procédé selon l'une quelconque des revendications 1 à 21, dans lequel la distance entre des points de grille adjacents le long d'au moins une dimension n'excède pas 7 µm.

23. Procédé selon la revendication 22, dans lequel la distance entre des points de grille adjacents le long d'au moins une dimension est inférieure à 4 µm, tel qu'entre 0,01 µm et 4 µm, préférablement 0,1 µm et 4 µm, plus préférablement entre 0,5 µm et 3,5 µm, p. ex. entre 1 µm et 3 µm.

24. Procédé selon la revendication 22 ou 23, dans lequel la distance entre des points de grille adjacents le long des deux dimensions n'excède pas 4 µm, préférablement entre 0,01 µm et 4 µm, préférablement entre 0,1 µm et 4 µm, plus préférablement entre 0,5 µm et 3,5 µm, tel qu'entre 1 µm et 3 µm.

25. Procédé selon l'une quelconque des revendications 1 à 24, dans lequel la structure comprend des saillies d'au moins deux différentes formes géométriques de la section transversale.

26. Procédé selon la revendication 25, dans lequel les saillies de différente géométrie de section transversale sont arrangées sur la grille régulière à deux dimensions dans un motif alternant.

27. Procédé selon l'une quelconque des revendications 1 à 26, dans lequel la structure comprend des saillies de différente superficie de section transversale.

28. Procédé selon la revendication 27, dans lequel les saillies sont des crêtes allongées avec différentes longueurs.

29. Procédé selon la revendication 28, dans lequel les crêtes allongées chacune présentent une largeur comprise entre 0,1 µm et 2 µm, préférablement entre 0,5 µm et 1,5 µm.

30. Procédé selon la revendication 28 ou 29, dans lequel la distance entre des crêtes allongées adjacentes est inférieure à 2 µm, préférablement 0,1 µm et 2 µm, préférablement 0,5 µm et 1,5 µm.

31. Procédé selon l'une quelconque des revendications 28 à 30, dans lequel les longueurs respectives des crêtes allongées est inférieures à 20 µm, préférablement inférieures à 10 µm, p. ex. entre 0,5 µm et 10 µm.

32. Procédé selon l'une quelconque des revendications 1 à 31, dans lequel les saillies sont situées sur des points de grilles de la grille régulière à deux dimensions si bien que seulement un sous-ensemble de points de grilles est couvert par des saillies.

33. Procédé selon l'une quelconque des revendications 1 à 32, dans lequel les saillies sont arrangées dans des lignes parallèles, la distance des centres entre des saillies adjacentes étant différente dans des lignes adjacentes.

34. Procédé selon l'une quelconque des revendications 1 à 33, dans lequel la section transversale latérale de l'une ou de plusieurs caractéristiques présente une forme définie par une circonférence et/ou une géométrie sélectionnée(s) parmi les formes suivantes: la forme circulaire, ronde, étoile, carrée, rectangulaire, hexagonale et polygonale ou une combinaison de ces formes.

35. Procédé selon l'une quelconque des revendications 1 à 34, dans lequel l'une ou plusieurs caractéristiques présentent une section transversale généralement carrée.

36. Procédé selon la revendication 35, dans lequel l'une ou plusieurs caractéristiques présentent une section transversale généralement circulaire, et l'une ou plusieurs caractéristiques présentent une section transversale généralement carrée.

37. Procédé selon l'une quelconque des revendications 1 à 36, dans lequel la dimension latérale de l'écartement maximale entre toute caractéristique et sa voisine la plus proche (d; Y) est dans au moins l'un des intervalles: entre environ 0,5 µm et 1,0 µm, entre environ 1 µm et 2 µm, entre environ 2 µm et 4 µm, entre environ 4 µm et 6 µm, entre environ 8 µm et 10 µm, entre environ 10 µm et 12 µm, entre environ 12 µm et 14 µm, entre environ 14 µm et 16 µm.

38. Procédé selon l'une quelconque des revendications 1 à 37, dans lequel la surface du matériau est **caractérisée par** une structure topographique périodique à l'échelle micrométrique, dont la dimension de pas latéral dans toute dimension latérale est sélectionnée parmi au moins l'un des intervalles: entre environ 1 µm et 2 µm, entre environ 2 µm et 4 µm, entre environ 4 µm et 6 µm, entre environ 6 µm et 10 µm, entre environ 10 µm et 16 µm, entre environ 16 µm et 20 µm, entre environ 20 µm et 24 µm.

39. Procédé selon l'une quelconque des revendications 1 à 38, dans lequel chacune des caractéristiques de ladite structure topographique présente une dimension verticale de hauteur/profondeur sélectionnée parmi au moins l'un des intervalles: d'entre environ 1 nm et 0,1 µm, d'entre environ 0,1 µm et 0,5 µm, d'entre environ 0,07 µm et 1,6 µm, d'entre environ 1,6 µm et 3,0 µm, d'entre environ 3 µm et 10 µm.

40. Procédé selon l'une quelconque des revendications 1 à 39, dans lequel le centre des caractéristiques de ladite structure topographique périodique est situé sur des points de grille d'une grille régulière et rectangulaire à deux dimensions avec des constantes de grille a et b, et où
a. la grille est une grille carrée, dans laquelle la constante de grille dans chaque direction (a=b) est dans un intervalle entre 2 et 12 µm, ou
b. la grille est rectangulaire avec une constante de grille (a) dans une première direction dans un intervalle entre 2 et 12 µm et avec une constante de grille (b) dans une deuxième direction dans un intervalle entre 1 et 6 µm, entre environ 6 µm et 10 µm, entre environ 10 µm et 16 µm, entre 16 µm et 20 µm, entre 20 µm et 24 µm.

41. Procédé selon l'une quelconque des revendications 1 à 40, dans lequel au moins une partie de ladite surface est revêtue de tantalum et/ou de titane.

42. Procédé selon l'une quelconque des revendications 1 à 41, dans lequel au moins quelques-unes des caractéristiques présentent une surface supérieure avec une structure topographique à l'échelle nanométrique.
